# EUROPEAN PATENT APPLICATION

(11) **EP 3 542 802 A1**
(43) Date of publication of application: **25.09.2019**
(21) Application number: 19157866.5
(22) Date of filing: 31.10.2014
(51) Int. Cl.: A61K 31/7105, C12N 15/117

(54) **MODIFIED RNA WITH DECREASED IMMUNOSTIMULATORY PROPERTIES**

(30) Priority: 01.11.2013 WO PCT/EP2013/003293
(62) Divisional of application: 14809289.3
(71) Applicant: CureVac AG, 72076 Tübingen (DE)
(72) Inventor: Schlake, Thomas, 72076 Tübingen (DE); Theß, Andreas, 72076 Tübingen (DE)
(74) Representative: Graf von Stosch Patentanwaltsgesellschaft mbH

(57) **Abstract**

The present invention provides a method for providing modified mRNAs of reduced immunogenicity and/or immunostimulatory capacity for use in protein replacement therapy. The invention further provides modified mRNAs and pharmaceutical compositions comprising the modified mRNAs according to the invention for use in protein replacement therapy.

## Description

### FIELD OF THE INVENTION

The present invention relates to a method for providing an mRNA with decreased immunostimulatory properties, which encodes at least one biologically active polypeptide or protein and its use in protein replacement therapy.

### BACKGROUND OF THE INVENTION

Therapeutic RNA molecules represent an emerging class of drugs. RNA-based therapeutics include messenger RNA (mRNA) molecules encoding antigens for use as vaccines. mRNA vaccines combine desirable immunological properties with the flexibility of genetic vaccines. One of several advantages of using mRNA for vaccination is that the same molecule not only provides an antigen source for the induction of an adaptive immune response, but can simultaneously bind to pattern recognition receptors such as Toll like receptors (TLRs) and thereby stimulate innate immunity. It has previously been reported that a two-component mRNA-based vaccine with dual activity induces balanced TLR-7 dependent adaptive immune responses and provides anti-tumor activity (Fotin-Mleczek et al., 2011. J. Immunother. 34(1):1-15). In addition, mRNA is considered to be a safer vector than DNA-based vectors because RNA cannot integrate into genomic DNA possibly leading to insertional mutagenesis.

mRNA molecules may also be used as therapeutics for replacement therapies, such as e.g. protein replacement therapies for substituting missing or mutated proteins such as growth factors or enzymes, in patients. However, successful development of safe and efficacious mRNA-based replacement therapies are based on different requirements compared to vaccines. When applying mRNA for protein replacement therapies, the mRNA should confer maximal expression of the protein of interest in terms of expression level and duration and minimal stimulation of the immune system to avoid general immune responses by the patient to be treated and specific immune responses against the administered mRNA molecule.

Whereas the inherent immunostimulatory property of mRNA is considered as a desirable feature for vaccines, this effect may cause undesired complications in replacement therapies. This is especially the case for the treatment of chronic diseases in which the mRNA therapeutic needs to be administered repeatedly over an extended period of time to patients. Although it has been demonstrated in animal studies that mRNA-encoded growth factor erythropoietin (EPO) can be successfully expressed *in vivo* resulting in biologically relevant increases of reticulocytes (Schlake et al., 2012. RNA Biol. 9(11):1319-30; Kariko et al., 2012. Mol. Ther. 20(5):948-53; Kormann et al., 2011. Nat. Biotechnol. 29(2):154-7), it has been recognized that the immunostimulating properties of the mRNA may potentially give rise to issues during therapy. Accordingly, such properties should be decreased.

Mammalian cells harbor a diverse set of nucleic acid-sensing pattern recognition factors (PRRs) receptors that recognize RNA by various recognition patterns (Review: Desmet et al., 2012. Nat. Rev. Immunol. 12(7):479-91). Toll-like receptors (TLRs) are the best studied group of pattern recognition factors (PRRs). Out of the 10 human TLRs four TLRs (TLR3, TLR7, TLR8 and TLR9) are nucleic acid sensors that recognize diverse pathogen-derived nucleic acids. Whereas TLR3, TLR7 and TLR8 recognize RNA, TLR9 binds to CpG motifs in DNA. TLR3, TLR7, TLR8 and TLR9 are intracellular TLRs and recognize nucleic acids that are taken up by the cell via endocytosis and transferred to endosomes. With the exception of TLR3, all nucleic acid-sensing TLRs use the adaptor protein MYD88 for downstream signaling to activate the transcription factors AP1 and NF- B leading to the expression of pro-inflammatory cytokines. These cytokines mediate the recruitment and activation of immune cells. TLR3 signaling depends on the TIR-domain-containing adaptor protein inducing IFN (TRIF) leading to the expression of pro-inflammatory cytokines as well as type I interferons (IFNs), which play a role in antiviral responses. For example, following TLR7 and TLR9 activation, plasmacytoid dendritic cells (pDCs) can produce large amounts of type I interferons (Desmet et al., 2012. Nat. Rev. Immunol. 12(7):479-91).

RIG-I-like receptors (RLRs) are members of the DExD/H-box helicase superfamily that act as cytosolic RNA sensors. Members of this family are retinoic acid-inducible gene I (RIG-I), melanoma differentiation-associated protein 5 (MDA5) and laboratory of genetics and physiology 2 (LGP2). RLRs are expressed broadly by immune and non-immune cells. The typical natural ligand of RIG-I is a short RNA with blunt-ended base-pairing and an uncapped 5' triphosphate end, but RIG-I has also been shown to bind to various double-stranded RNA (dsRNA) and single-stranded RNA (ssRNA) ligands. MDA5 generally binds to long dsRNA molecules but is also involved in the discrimination of self and non-self RNAs based on the ribose 2'-O-methylation status of the cap structure (Züst et al., 2011. Nat. Immunol. 12(2):137-43, PMID 21217758). In addition, RIG-I and MDA5 can be activated by self RNAs that are cleaved by RNAse L. RNase L is a ribonuclease that is induced in response to type I interferons and degrades all the RNA within the cell. RLR signaling depends on the adaptor IFNB-promotor stimulator 1 (IPS1) leading to the activation of transcription factors IRF1, IRF3, IRF7 and NF-κB and subsequently to the expression of type I IFNs and pro-inflammatory cytokines (Broz et al., 2013. Nat. Rev. Immunol. 13(8):551-65).

2'-5' oligoadenylate synthetases (OASs) and the protein kinase regulated by RNA (PKR) are both interferon-induced, dsRNA-dependent enzymes located in the cytosol that play important roles in mediating the antiviral effects of the interferons. PKR is a protein serine/threonine kinase that acquires enzymatic activity following autophosphorylation, a process mediated by dsRNA. Activation of PKR allows the kinase to phosphorylate its natural substrate, the alpha subunit of eukaryotic protein synthesis initiation factor-2 (EIF2-alpha), leading to the inhibition of protein synthesis. The 2'-5' oligoadenylate synthetases are also activated by dsRNA and subsequently polymerize ATP into 2'-5'-linked oligoadenylates (2'-5' (A)) of various lengths, which function as specific activators of a latent endoribonuclease, RNase L. Once activated by 2'-5' (A), RNase L degrades viral and cellular RNAs resulting in the inhibition of protein synthesis. Both enzymes, PKR and 2'-5' oligoadenylate synthetase, are activated by ssRNA and dsRNA that possess extensive secondary structure (Sharp et al., 1999. Virology 257(2):303-13).

Accordingly, RNA molecules may exert an unspecific immune response in patients, which should be avoided or at least reduced. Several approaches were described for decreasing the immunostimulatory properties of mRNA including the incorporation of naturally occurring modified nucleosides into *in vitro* transcribed mRNA. The purification of such mRNAs is expected to remove contaminants or double-stranded RNA. Alternatively, the synthesis and use of novel non-naturally occurring modified nucleosides with decreased immunostimulatory potential was envisaged as well.

Kariko et al. demonstrated that different mRNAs stimulated the secretion of cytokines, such as TNF, by human dendritic cells (DCs) to various extents, an effect that was attributed to the engagement of TLR3, 7 and 8 (Kariko et al., 2005. Immunity 23(2):165-75). Whereas synthetic dsRNA, *in vitro* transcribed RNA as well as bacterial and mitochondrial RNA induced strong cytokine secretion, cytoplasmic RNA from mammalian cells stimulated DCs to a much lower extent. In addition, it was shown that RNA signals through human TLR3, TLR7, and TLR8. Incorporation of modified nucleosides such as 5-methylcytidine (m5C), N6-methyladenosine (m6A), 5-methyluridine (m5U), 2-thiouridine (s2U), or pseudouridine (ψ), however, ablated this activity. Dendritic cells (DCs) treated with modified RNA expressed significantly less cytokines and activation markers than those treated with unmodified RNA. The authors concluded that nucleoside modifications suppress the potential of RNA to activate DCs. Therefore, it was suggested that mRNAs containing naturally occurring modified nucleosides may be used in clinical applications due to their reduced immunostimulating properties (Kariko et al., 2007. Curr. Opin. Drug Discov Devel. 10(5):523-32; WO2007024708A1).

Further work demonstrated that *in vitro* generated mRNA containing uridine activated RNA-dependent protein kinase (PKR), which then phosphorylated translation initiation factor 2-alpha (eIF-2), and inhibited translation. In contrast, *in vitro* transcribed mRNAs containing pseudouridine as a modified nucleoside activated PKR to a lesser degree, while translation of pseudouridine-containing mRNAs was not repressed (Anderson et al., 2010. Nucleic Acids Res. 38(17):5884-92).

In addition, the Kariko group showed that *in vitro* transcribed, unmodified RNA activates 2'-5' oligoadenylate synthetase (OAS), induced RNase L-mediated ribosomal RNA (rRNA) cleavage and was rapidly cleaved by RNase L. In contrast, RNA containing modified nucleosides activated OAS less efficiently and induced limited rRNA cleavage, thus showing the role of nucleoside modifications in suppressing immune recognition of RNA (Anderson et al., 2011. Nucleic Acids Res. 39(21):9329-38).

Purification of *in vitro* transcribed mRNA by high performance liquid chromatography (HPLC) has been shown to reduce immune activation by transfected mRNA. It was reported that contaminants, including dsRNA, in nucleoside-modified *in vitro* transcribed RNA were responsible for innate immune activation and that their removal by high performance liquid chromatography (HPLC) resulted in mRNA that did not induce the production of IFNs and inflammatory cytokines. It was translated at 10- to 1000-fold greater levels in primary cells. Although unmodified mRNAs were translated significantly better following purification, they still induced significant levels of cytokine secretion (Kariko et al., 2011. Nucleic Acids Res. 39(21):e142). Treatment of mice with pseudouridine modified mRNA coding for erythropoietin (EPO) gave rise to higher protein expression and stronger biological effects compared to unmodified mRNA. Moreover, the modified RNA did not induce detectable levels of interferon and anti-EPO antibodies in plasma. In addition, it was shown that intraperitoneal injection of EPO mRNA into macaques increased serum EPO levels (Kariko et al., Mol. Ther. 20(5):948-53).

By another study, using a combination of different nucleotide modifications, enhanced expression and reduced immunostimulation of an EPO-encoding mRNA was demonstrated in mice. Furthermore, in a mouse model of a lethal congenital lung disease caused by the lack of surfactant protein B (SP-B), the application of an aerosol of modified SP-B mRNA to the lung showed a therapeutic effect (Kormann et al., 2011. Nat. Biotechnol. 29(2):154-7; WO2011012316).

The induction of induced pluripotent stem cells (iPSCs) was reported by transfection of human cells with synthetic modified mRNA. Improved expression and reduced immunostimulation, indicated by lower expression of interferon-regulated genes, were observed with base-modified mRNAs containing 5-methylcytidine and pseudouridine (Warren et al., 2010. Cell Stem Cell. 7(5):618-30; WO2011130624; WO2011071931).

WO2013/052523 reports novel modified nucleosides, nucleotides and nucleic acids which can exhibit a reduced innate immune response when introduced into cells. However, any approach to reduce innate immune stimulation based on an mRNA containing non-natural modified nucleosides is less desired. Any such modification, which does not usually occur in patients, bears the risk of undesired side effects.

In view of the above, there is a continued need for novel methods to reduce the immunostimulatory properties of mRNA while maintaining efficient protein expression and a good safety profile avoiding any risk of undesired side effects.

### SUMMARY OF THE INVENTION

The present inventors have identified an appropriate method to modulate, preferably decrease, the immunogenicity and/or immunostimulatory capacity of a target mRNA (immune response against an mRNA), which encodes at least one biologically active polypeptide or protein, by preferably increasing the cytosine-content (C-content) of the coding region encoding the at least one biologically active polypeptide or protein and optionally exchanging additionally relatively rare codons in the target wild type sequence by relatively frequent codons that code for the same amino acid.

According to a first embodiment, the method for targeted modulation, preferably reduction of the immune response against an mRNA coding for at least one biologically active polypeptide or protein of the present invention comprises the steps of identifying a target mRNA wild type sequence coding for the biologically active polypeptide or protein and modifying said mRNA by replacing at least 70% of the codons of the wild type sequence, which are "cytosine content optimizable", by codons with a higher cytosine content, thereby increasing the cytosine-content of the mRNA such that the cytosine-content of the coding region of the modified mRNA (coding for the polypeptide or protein) is larger than the cytosine-content of the coding region of the wild type mRNA (coding for the at least one polypeptide or protein), whereby the encoded amino acid sequence remains unchanged compared to the wild type (amino acid) sequence.

In a preferred embodiment of the present invention, the modified target mRNA is modified such that at least 10%, 20%, 30%, 40%, 50%, 60%, 70% or 80%, or at least 90% of the theoretically maximal cytosine-content or even a maximal cytosine-content is achieved.

In further preferred embodiments, at least 10%, 20%, 30%, 40%, 50%, 60%, 70%; 80%, 90% or even 100% of the codons of the target mRNA wild type sequence, which are "cytosine content optimizable" are replaced by codons with a higher cytosine-content as present in the wild type sequence.

In a further preferred embodiment, some of the codons of the wild type coding sequence may additionally be modified such that a codon for a relatively rare tRNA in the cell is exchanged by a codon for a relatively frequent tRNA in the cell, provided that the substituted codon for a relatively frequent tRNA carries the same amino acid as the relatively rare tRNA of the original wild type codon. Preferably, all of the codons for a relatively rare tRNA are replaced by a codon for a relatively frequent tRNA in the cell, except codons encoding amino acids, which are exclusively encoded by codons not containing any cytosine, or except for glutamine (Gln), which is encoded by two codons each containing the same number of cytosines.

In a further preferred embodiment of the present invention, the modified target mRNA is modified such that at least 80%, or at least 90%of the theoretically maximal cytosine-content or even a maximal cytosine-content is achieved by means of codons, which code for relatively frequent tRNAs in the cell, wherein the amino acid sequence remains unchanged.

Accordingly, the above objects of the present invention are solved by a method, which provides for a modified target mRNA encoding at least one biologically active polypeptide or protein with decreased immunogenicity and/or immunostimulatory capacity compared to the wild type sequence, wherein the C-content of the coding region of the modified target mRNA is typically increased by at least 10%, preferably by at least 12.5%, more preferably by at least 15% based on the cytosine-content of the polypeptide or protein coding region of the wild type mRNA.

In a specific embodiment, the present invention provides for a method, in which, additionally to the increase in cytosine-content, some codons, preferably all codons of the wild type sequence, which are not cytosine-content optimizable or which do not code for glutamine, and which reflect a relatively rare codon in the cell, are replaced by codons, which code for a relatively frequent tRNA in the cell, which carries the same amino acid as the relatively rare tRNA.

Preferably, the codon adaptation index (CAI) of the region of the immunologically modulated mRNA coding for the polypeptide or protein according to the invention is increased by at least 0.05, preferably by at least 0.1, preferably by at least 0.125, most preferable by at least 0.15 as compared to the CAI of the wild type coding region of the mRNA encoding for the polypeptide or protein. Accordingly, the modified mRNA displays preferably an increased level of expression compared to the wild type mRNA.

The method according to the present invention may further comprise the step of assaying the immunogenicity and/or immunostimulatory capacity of the modified target mRNA coding for the at least one polypeptide or protein. Preferably, the immunogenicity and/or immunostimulatory capacity of the modified target mRNA encoding at least one biologically active polypeptide or protein is assayed by transfecting peripheral blood monocytes (PBMCs) in vitro with the inventive modified target mRNA, culturing the cells for at least 8 hours, preferably for at least 12 hours, more preferably for at least 20 hours and determining the amount of pro-inflammatory cytokines in the cell supernatant. The result of such an assay may be compared - in a further optional step - with the result of parallel experiments carried out for the underlying wild type (wt) sequence.

According to the invention, the target mRNA coding for the at least one biologically active polypeptide or protein of lower immunogenicity and/or immunostimulatory capacity compared to the wild type sequence is selected from all of the modified target mRNAs obtainable by a method of the invention. Optionally, the inventive method according to the above embodiments may be reiterated to further decrease the immunogenicity and/or immunostimulatory capacity of the target mRNA, which encodes at least one biologically active polypeptide or protein. Accordingly, alternative modified target mRNAs exhibiting the structural properties of the invention may be produced and tested.

According to one embodiment of the present invention, the targeted modulation of the immune response against an mRNA encoding at least one biologically active polypeptide or protein is carried out by way of executing at least one algorithm of the cytosine-optimization on a computer with the aid of suitable software.

The present invention also provides for a modified mRNA of reduced immunogenicity and/or immunostimulatory capacity encoding at least one biologically active polypeptide or protein according to one or more embodiments of the present invention, wherein the modified mRNA is obtained by means of chemical or biological synthesis. Preferably, the modified mRNA is obtained by *in vitro* transcription, preferably by bacteriophage polymerase-mediated *in vitro* transcription, e.g. by Sp6 polymerase *in vitro* transcription and/or T3 polymerase-mediated *in vitro* transcription, preferably by T7 polymerase-mediated *in vitro* transcription.

The present invention also provides a modified mRNA of reduced immunogenicity and/or immunostimulatory capacity encoding at least one biologically active polypeptide or protein obtainable by methods according to the invention.

In this context, it is particularly preferred that the modified mRNA of reduced immunogenicity and/or immunostimulatory capacity encoding at least one biologically active polypeptide or protein according to the invention has at least 10%, 20% or at least 30% lower immunogenicity and/or immunostimulatory capacity as compared to the respective wild type mRNA.

The modified mRNA of reduced immunogenicity and/or immunostimulatory capacity according to the present invention is typically characterized by a lower affinity, e.g. at least decreased by 20% to one or more, to TLR3, TLR7, TLR8, PKR, MDA5, RIG-I, LGP2 or 2'-5'-oligoadenylate synthetase, as compared to the wild type mRNA encoding the at least one polypeptide or protein.

Furthermore, the inventive modified mRNA according to one or more of the above embodiments comprises a 5' CAP structure, and/or at least one 3'- and/or 5'-UTR (untranslated region) and/or a polyA-tail of at least 60 nucleotides, more preferably of at least 70 nucleotides and/or a 3' stabilizing sequence.

Accordingly, the present invention also provides a pharmaceutical composition, which comprises a modified mRNA according to one or more of the above embodiments, which optionally comprises one or more pharmaceutically acceptable excipients, carriers, diluents and/or vehicles.

More specifically, the present invention provides a pharmaceutical composition according to the above embodiment or the modified mRNA according to one or more of the above embodiments for use in the treatment of protein replacement therapy, preferably for use in the treatment of e.g. hereditary or endocrinological diseases.

Furthermore, the present invention provides a method of treating a subject in need of protein replacement therapy, the method comprising administering to a subject in need thereof a pharmaceutically effective amount of the pharmaceutical composition according to one or more of the above embodiments or an effective amount of the modified mRNA according to one or more of the above embodiments.

More specifically, the present invention provides a method according to the above embodiment, wherein the disease to be treated by protein replacement therapy is selected from the group consisting of hereditary or endocrinological disorders, such as e.g. amino acid metabolism disorders, carbohydrate metabolism disorders, cholesterol biosynthesis disorders, fatty acid oxidation defects and fat metabolism disorders, lactic acidosis, glycogen storage diseases, mitochondrial disorders, organic acid disorders, urea cycle disorders, lysosomal storage disease disorders.

More preferably, the present invention provides a method for expressing a biologically active polypeptide or protein in a tissue *in vivo,* the method comprising contacting the patient with a pharmaceutical composition according to one or more of the above embodiments of the invention, or contacting the patient with the inventive modified mRNA according to any one of the above embodiments of the invention, wherein administering the pharmaceutical composition or the modified mRNA results in a reduced innate immune response by the patient relative to a patient, preferably the same patient, contacted with the wild type mRNA molecule encoding the same polypeptide or protein. Optionally, the level of mRNA expression *in vivo* is increased by the modified target mRNA of the invention as compared to the wild type mRNA.

According to a further embodiment, the present invention provides for a modified mRNA that codes for at least one biologically active polypeptide or protein, wherein the cytosine-content of the coding region of the modified mRNA is larger than the cytosine-content of the coding region of the wild type mRNA coding for the polypeptide or protein, whereby the encoded amino acid sequence is unchanged compared to the wild type sequence. In this context, it is preferred that, if no cytosine is present in any of the at least one codon coding for the amino acid, at least one codon of the wild type sequence that codes for a relatively rare tRNA in the cell is exchanged for a codon that codes for a relatively frequent tRNA in the cell that carries the same amino acid as the relatively rare tRNA.

### BRIEF DESCRIPTION OF THE DRAWINGS

The present invention is described by the figures and examples below, which are used only for illustration purposes and are not meant to limit the scope of the invention. Owing to the description and examples, further embodiments are likewise included in the invention that are accessible to the skilled person.

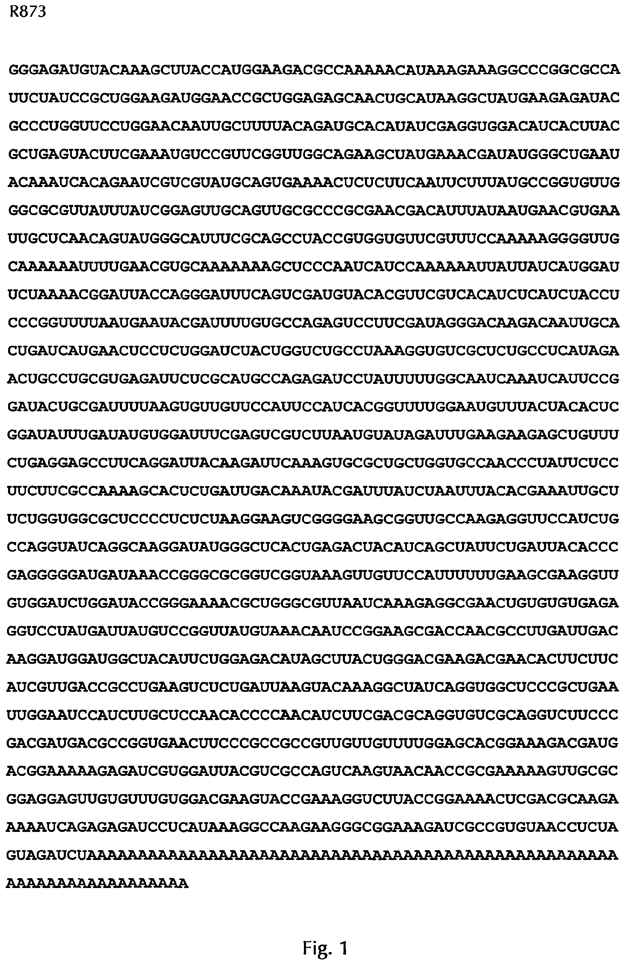

- **Figure 1:**: Wildtype mRNA sequence R873 coding for *Photinus pyralis* luciferase (PpLuc), which corresponds to SEQ ID NO:1. The mRNA was *in vitro* transcribed from a vector containing a T7 promoter followed by a sequence coding for *Photinus pyralis* luciferase (PpLuc(wt), and a poly(A) sequence of 70 adenosine nucleotides (A70). This sequence was subsequently used as a target sequence for modulating the immunogenicity and/or immunostimulatory capacity according to the inventive method.

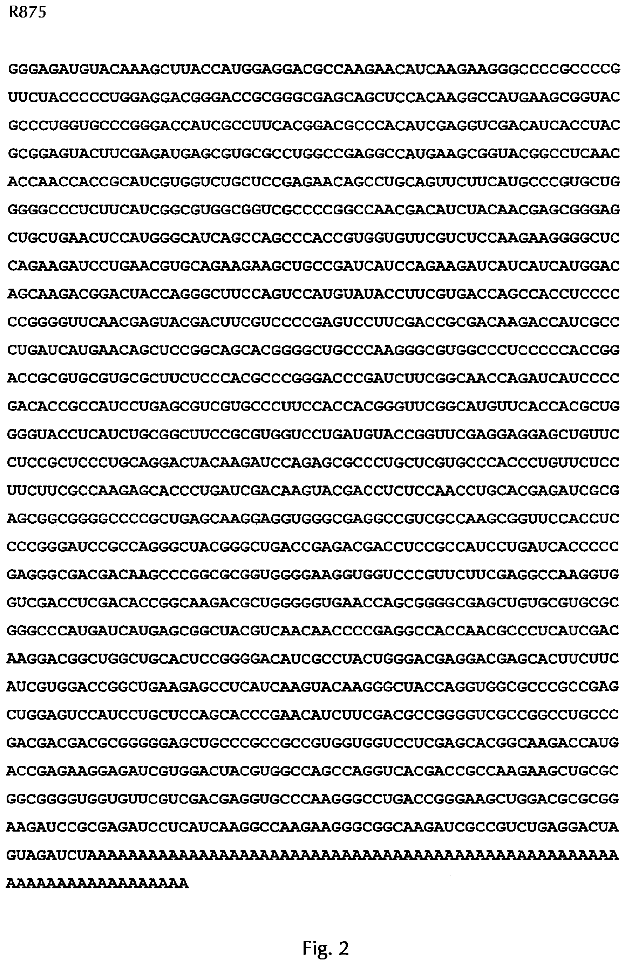

- **Figure 2:**: G/C-enriched mRNA sequence R875 which is obtained by G/C enrichment only coding for *Photinus pyralis* luciferase (PpLuc), which corresponds to SEQ ID NO:2. R875 was obtained by G/C enrichment of the sequence R873.

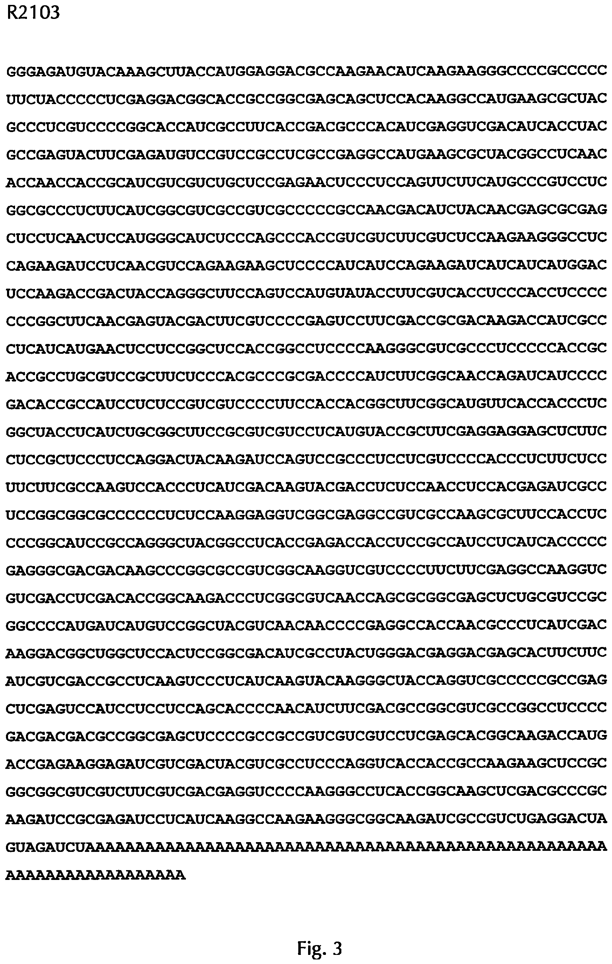

- **Figure 3:**: C-enriched mRNA sequence R2103 obtained by G/C enrichment and subsequent C-enrichment coding for *Photinus pyralis* luciferase (PpLuc), which corresponds to SEQ ID NO: 3. R2103 was obtained by C-enrichment of the sequence R873.

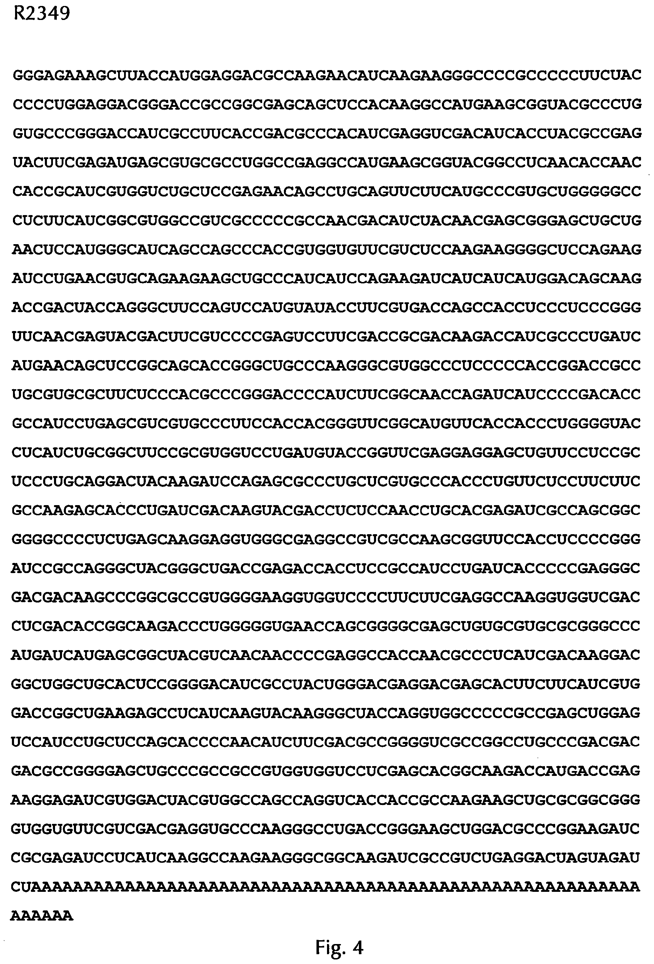

- **Figure 4:**: G/C-enriched mRNA sequence R2349 coding for Photinus pyralis luciferase (PpLuc), corresponding to SEQ ID NO:4. The mRNA was *in vitro* transcribed from a vector containing a T7 promoter followed by a G/C-enriched sequence coding for Photinus pyralis luciferase (PpLuc(GC)III) and a sequence of 64 adenosine nucleotides (A64) poly(A) sequence.

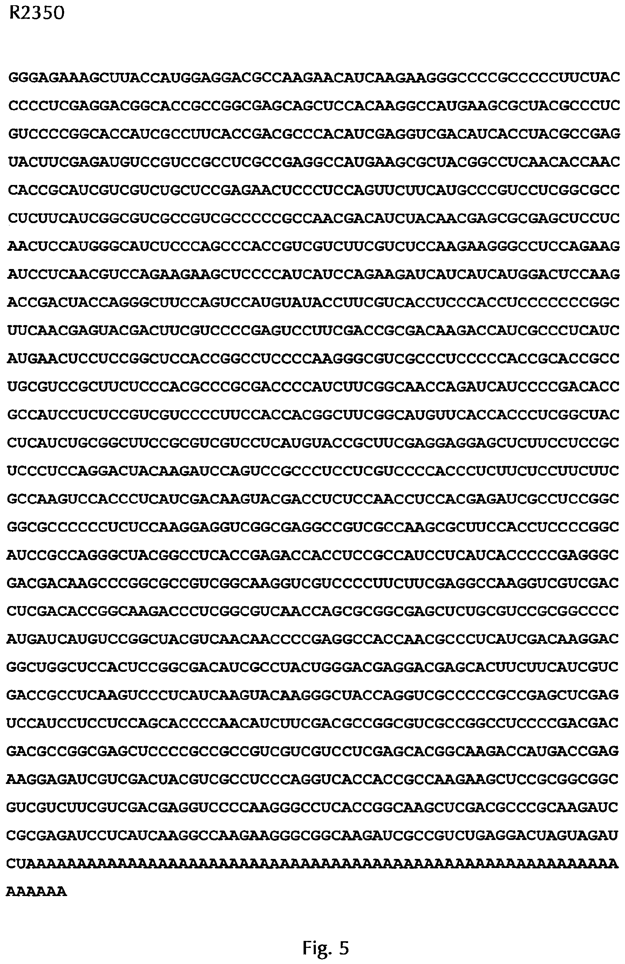

- **Figure 5:**: C-enriched mRNA sequence of R2350 coding for Photinus pyralis luciferase (PpLuc), which corresponds to SEQ ID NO:5. The template for *in vitro* transription was obtained by modifying the vector comprising the G/C-enriched sequence by replacing the GC-optimized coding sequence of PpLuc(GC)III (Figure 4) by a C-enriched sequence. mRNA obtained from this vector by *in vitro* transcription is designated "PpLuc(GC)III - A64" (R2350).

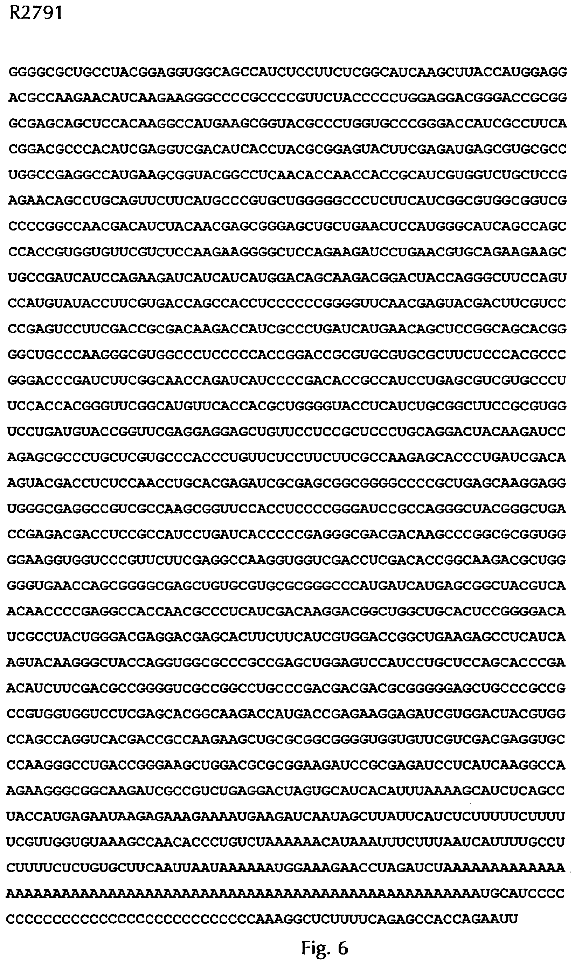

- **Figure 6:**: G/C-enriched mRNA sequence R2791 coding for Photinus pyralis luciferase (PpLuc), corresponding to SEQ ID NO:6. The vector used for *in vitro* transcription comprised a 5'-TOP-UTR derived from the ribosomal protein 32L, followed by a stabilizing sequence derived from the albumin-3'-UTR, a stretch of 64 adenosine nucleotides (poly(A)-sequence), a stretch of 30 cytosines (poly(C)-sequence), and a histone stem loop.

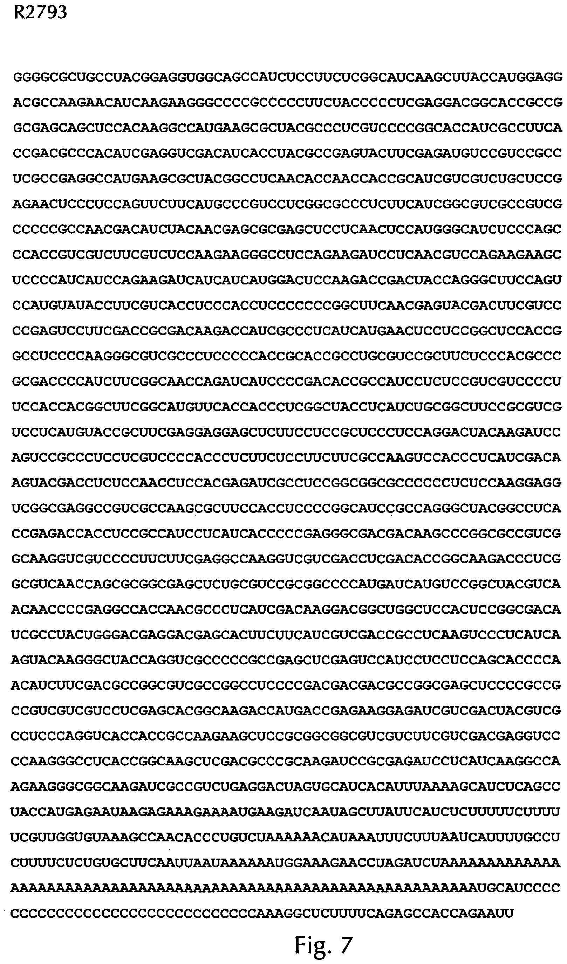

- **Figure 7:**: C-enriched mRNA sequence of R2793 coding for Photinus pyralis luciferase (PpLuc), corresponding to SEQ ID NO:7. The vector used for *in vitro* transcription comprises a 5'-TOP-UTR derived from the ribosomal protein 32L, followed by a stabilizing sequence derived from the albumin-3'-UTR, a stretch of 64 adenosines (poly(A)-sequence), a stretch of 30 cytosines (poly(C)-sequence), and a histone stem loop.

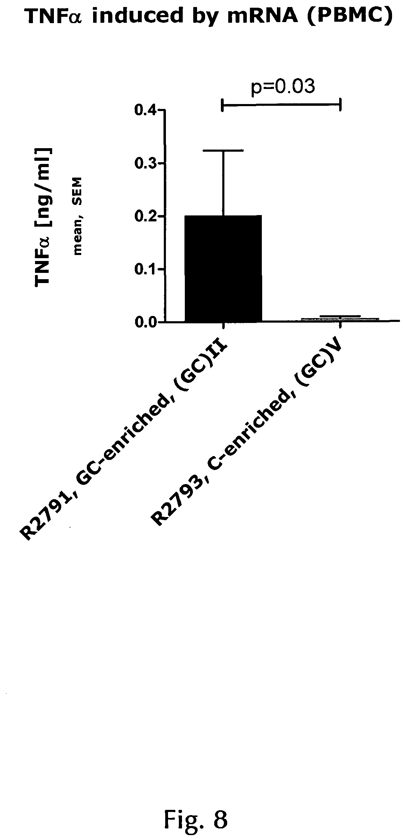

- **Figure 8:**: TNFa secretion of PBMCs treated with R2793 and R2791 mRNAs. Human PBMCs were treated with 10 µg/ml of GC- or C-enriched mRNA for 20 hours and the TNFa concentration was determined in the supernatant by ELISA as described in Example 2.
As can be seen, the treatment with C-enriched mRNA (R2793) results in significantly less TNFa secretion than treatment with GC-enriched mRNA (R2791). The statistical significance was assessed by the Mann Whitney test (p = 0.03).

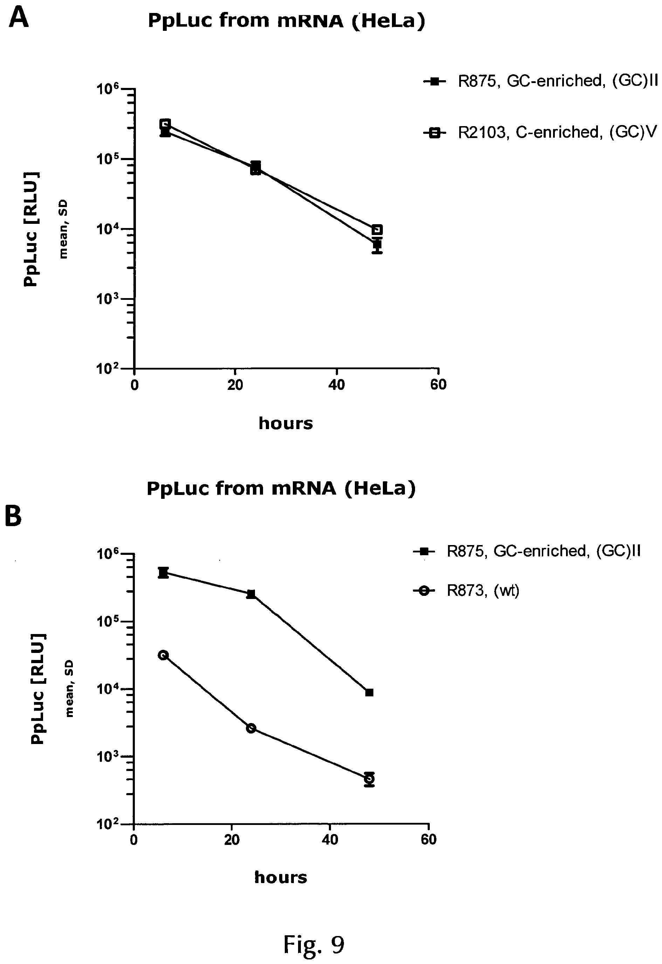

- **Figure 9:**: Luciferase activity expressed by wild type or modified mRNAs. Both RNAs were separately transfected into HeLa cells and luciferase activity (relative light units, RLU) was measured 6h, 24h and 48h after transfection as described in Example 2.
(A) The luciferase activity of GC-enriched mRNA (R875) and C-optimized mRNA (R2103) was comparable both in terms of peak level and kinetics indicating a comparable expression level of the transfected mRNAs as a function of time.
(B) The luciferase activity of GC-enriched mRNA (R875) was much higher than that of the wild type construct (R873) indicating a significantly increased expression level of the modified G/C enriched mRNA.

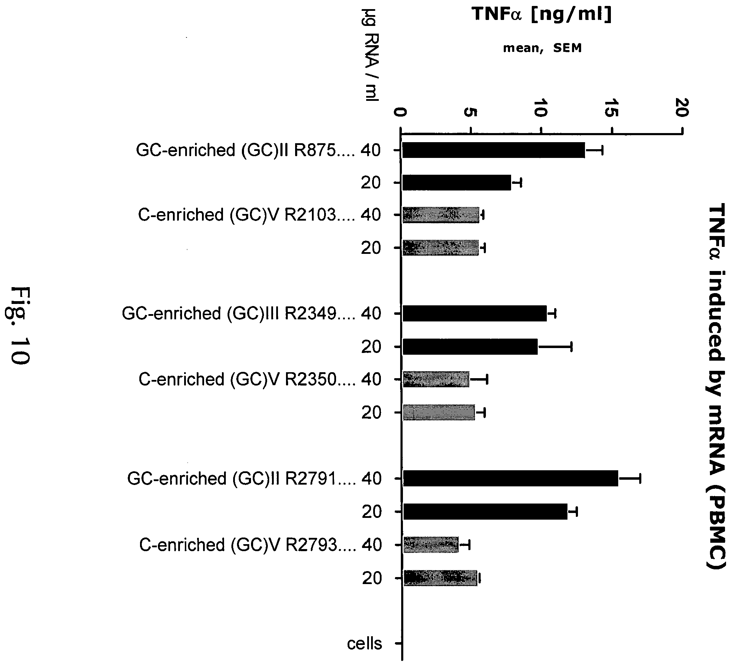

- **Figure 10:**: Dose-response relationship for TNFa secretion of human PBMCs treated with different modified mRNAs.
Human PBMCs were treated with different concentrations of mRNAs (40 and 20 µg/ml) for 20 hours. The TNFa concentration as a parameter indicating the immune response evoked by the transfected mRNA in the immunologically competent PBMCs was determined by ELISA in the supernatants as described in Example 3. Treatment with C-optimized mRNA results in less TNFα secretion than treatment with GC-optimized mRNA. The mean and standard error of mean of triplicates are shown.

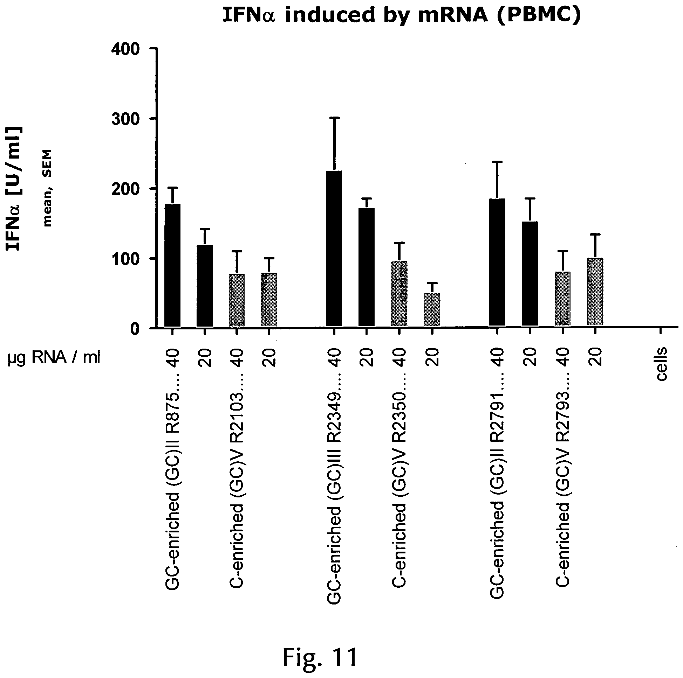

- **Figure 11:**: Dose-response relationship for IFNa secretion of human PBMCs treated with various modified mRNAs.
Human PBMCs were treated with different concentrations of mRNAs (40 and 20 µg/ml) as indicated for 20 hours. The IFNa concentration was determined by ELISA in the supernatants as described in Example 3. Transfection of the PBMCs with C-optimized mRNA results in less IFNα secretion than transfection with GC-optimized mRNA. The mean and standard error of mean of triplicates are shown.
- **Table 1:**: List of luciferase constructs used for the production of the wild type (R873) and G/C- or C-enriched modified mRNA constructs used.
- **Table 2:**: Summary of the nucleotide composition and codon usage of the constructs used in the present Examples.

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered as disclosed by the description of the present application, unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of".

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

The present invention is based on the surprising finding that the immune response against a target mRNA, which encodes at least one biologically active polypeptide or protein, can be modulated, i.e. reduced, by the inventive method of increasing the cytosine-content of the coding region of the mRNA. The inventive method hereby comprises the step of identifying a target wild type sequence, which encodes for at least one biologically active peptide, polypeptide or protein. As a second step, the target wild type sequence is modified, whereby at least 70% of the codons, or at least 80% of the codons, or more preferably at least 90%, or most preferably 100% of the wild type codons of the coding region, which are cytosine content-optimizable are modified such that the overall cytosine-content of the region of the modified mRNA coding for the peptide, polypeptide or protein is increased over the cytosine-content of the coding region of the wild type mRNA coding for the at least one peptide, polypeptide or protein. By that modification, the amino acid sequence encoded by the modified mRNA is unchanged compared to the wild type sequence.

Due to the naturally occuring degeneracy of the genetic code, more than one codon may encode a particular amino acid. Accordingly, 18 out of 20 naturally occuring amino acids are encoded by more than 1 codon (with Tryp and Met being an exception), e.g. by 2 codons (e.g. Cys, Asp, Glu), by three codons (e.g. Ile), by 4 codons (e.g. Al, Gly, Pro) or by 6 codons (e.g. Leu, Arg, Ser). However, not all codons encoding the same amino acid are utilized equally frequent under *in vivo* conditions. Depending on each single organism, a typical codon usage profile is established.

The term "cytosine content-optimizable codon" as used within the context of the present invention refers to codons, which exhibit a lower amount of cytosines than other codons coding for the same amino acid. Accordingly, any wild type codon, which may be replaced by another codon coding for the same amino acid and exhibiting a higher number of cytosines within that codon, is considered to be cytosine-optimizable (C-optimizable). Any such substitution of a C-optimizable wild type codon by the specific C-optimized codon within a wild type coding region increases its overall C-content and reflects a C-enriched modified mRNA sequence. A C-maximized mRNA sequence contains C-optimized codons for all potentially C-optimizable codons. Accordingly, 100% or all of the theoretically replaceable C-optimizable codons are under such conditions actually replaced by C-optimized codons over the entire length of the coding region.

Within the context of the present invention the preferred cell is a mammalian cell, more preferably a human cell. The codon usage frequency for the individual codons is provided in Table 2 of the appended examples. The term "coding region" as used in the present invention, i.e. the region in which the cytosine-content is increased by the use of cytosine-content optimized codons, corresponds to that portion of an RNA, such as, e.g. an mRNA, that codes for a peptide, polypeptide or protein. The coding region in mRNA may be bounded by the five prime untranslated region (5' UTR) and the three prime untranslated region (3' UTR).

Within the present invention, cytosine-content optimizable codons are codons, which contain a lower number of cytosines than other codons coding for the same amino acid. Any of the codons GCG, GCA, GCU codes for the amino acid Ala, which may be exchanged by the codon GCC encoding the same amino acid, and/or
the codon UGU that codes for Cys may be exchanged by the codon UGC encoding the same amino acid, and/or
the codon GAU which codes for Asp may be exchanged by the codon GAC encoding the same amino acid, and/or
the codon that UUU that codes for Phe may be exchanged for the codon UUC encoding the same amino acid, and/or
any of the codons GGG, GGA, GGU that code Gly may be exchanged by the codon GGC encoding the same amino acid, and/or
the codon CAU that codes for His may be exchanged by the codon CAC encoding the same amino acid, and/or
any of the codons AUA, AUU that code for Ile may be exchanged by the codon AUC, and/or
any of the codons UUG, UUA, CUG, CUA, CUU coding for Leu may be exchanged by the codon CUC encoding the same amino acid, and/or
the codon AAU that codes for Asn may be exchanged by the codon AAC encoding the same amino acid, and/or
any of the codons CCG, CCA, CCU coding for Pro may be exchanged by the codon CCC encoding the same amino acid, and/or
any of the codons AGG, AGA, CGG, CGA, CGU coding for Arg may be exchanged by the codon CGC encoding the same amino acid, and/or
any of the codons AGU, AGC, UCG, UCA, UCU coding for Ser may be exchanged by the codon UCC encoding the same amino acid, and/or
any of the codons ACG, ACA, ACU coding for Thr may be exchanged by the codon ACC encoding the same amino acid, and/or
any of the codons GUG, GUA, GUU coding for Val may be exchanged by the codon GUC encoding the same amino acid, and/or
the codon UAU coding for Tyr may be exchanged by the codon UAC encoding the same amino acid.

In any of the above instances, the number of cytosines is increased by 1 per exchanged codon. Exchange of all non C-optimized codons (corresponding to C-optimizable codons) of the coding region results in a C-maximized coding sequence. According to the invention at least 70% of the non C-optimized codons are replaced by C-optimized codons of the wild type sequence are replaced by C-optimized codons, preferably at least 80%, more preferably at least 90% within the coding region.
It may be preferred that for some amino acids the percentage of C-optimizable codons replaced by C-optimized codons is less than 70%, while for other amino acids the percentage of replaced codons is higher than 70% to meet the overall percentage of C-optimization of at least 70% of all C-optimizable wild type codons of the coding region. Preferably, in the C-optimized mRNAs of the invention, at least 50% of the C-optimizable wild type codons for any given amino acid are replaced by C-optimized codons, e.g. any modified C-enriched mRNA preferably contains at least 50% C-optimized codons at C-optimizable wild type codon positions coding for any single of the above mentioned amino acids Ala, Cys, Asp, Phe, Gly, His, Ile, Leu, Asn, Pro, Arg, Ser, Thr, Val and Tyr, preferably at least 60%.

According to the inventive method, codons coding for amino acids, which are not cytosine content-optimizable and which are, however, encoded by at least two codons, may be used without any further selection process. However, in a preferred embodiment of the invention the codon of the wild type sequence that codes for a relatively rare tRNA in the cell, e.g. a human cell, is exchanged for a codon that codes for a relatively frequent tRNA in the cell, whereby both code for the same amino acid. Accordingly, the relatively rare codon GAA coding for Glu may be exchanged by the relative frequent codon GAG coding for the same amino acid, and/or the relatively rare codon AAA coding for Lys may be exchanged by the relative frequent codon AAG coding for the same amino acid, and/or
the relatively rare codon CAA coding for Gln is exchanged for the relative frequent codon CAG encoding the same amino acid.

In accordance with the present invention, the amino acids Met (AUG) and Trp (UGG), which are encoded by only one codon each, remain unchanged. Stop codons are not cytosine-content optimized, however, the relatively rare stop codons *amber, ochre*(UAA, UAG) may be exchanged by the relatively frequent stop codon *opal*(UGA)*.*

The substitutions listed above may obviously be used individually but also in all possible combinations in order to optimize the cytosine-content of the modified mRNA compared to the wild type mRNA sequence.

Accordingly, the region of the modified mRNA coding for the polypeptide or protein is changed compared to the coding region of the polypeptide or protein of the wild type mRNA in such a way that an amino acid encoded by at least two or more codons, of which one comprises one additional cytosine, such a codon may be exchanged by the C-optimized codon comprising one additional cytosine, whereby the amino acid is unaltered compared to the wild type sequence.

In a preferred embodiment, the modified mRNA has - in addition to an increased C-content as defined above - an increased guanosine-content (G-content). In analogy to the modifications described above, which result in an increased C-content, corresponding modifications may additionally be introduced, which result in a higher G-content of the modified mRNA. Therein, a codon in the mRNA, which is guanosine-content optimizable, is replaced by a codon with a higher guanosine-content. Generally, the definitions and explanations provided above with respect to cytosine-optimizable codons and increased C-content apply to guanosine-optimizable codons and G-content in analogous manner. In the context of the present invention, guanosine-optimizable codons are codons, which contain a lower number of guanosines than other codons that are encoding the same amino acid.

In a further preferred embodiment, the modified mRNA is modified such that at least 10%, 20%, 30%, 40%, 50%, 60%, 70%, 80% or 90%, preferably at least 70% of the codons, which are not eligible for C-optimization, but which are guanosine-content optimizable, are replaced by a codon with a higher guanosine content, thus increasing the G-content of the modified mRNA.

Alternatively, the modified mRNA may be obtained by the following approach:
As a first step, its G/C content may be increased, e.g. by substituting wild type codons exhibiting a lower content of G and C nucleotides. At least 70%, more preferably at least 80%, more preferred at least 90%, or most preferred 100% of all wild type codons, which are G/C optimizable, may be replaced by that approach. G/C optimization is carried out as disclosed in WO2002098443 A2. As a second step, the G/C-enrichment or maximization is followed by a step of further C-optimization. Accordingly, the G/C optimized codons and/or the unaltered codons within the coding region are C-optimized by selecting codons, which exhibit a higher number of cytsines as compared to the codons occurring in the G/C-enriched mRNA. At least 70%, more preferably at least 80%, more preferably at least 90%, most preferred 100% of those GC-optimized and unaltered codons, which are C-optimizable, are replaced by that second step. The second step coincides with the first step (as described above for the embodiment starting with C-enrichment), but starts from a codon pattern, which is different from the wild type codon pattern. As a result, G/C maximization and subsequent C-maximization over the entire length of the coding region usually results in the same coding sequence as the alternative embodiment starting with C-maximization and subsequent G-maximization as described above.

According to a further embodiment of the present invention, the cytosine content of the coding region of the target mRNA coding for the polypeptide or protein is modified such that a maximal cytosine-content is achieved by introduction of codons, which code for tRNAs, which are relatively frequent within that particular cell, e.g. such as a mammalian cell, more specifically a human cell.

According to the inventive method for targeted modulation, preferably reduction, of the immune response against an mRNA, the cytosine content of the modified mRNA, which encodes at least one biologically active polypeptide or protein, is increased by at least 1 - 15%, preferably 5 - 10%, preferably by at least 10 - 15%, preferably by at least 10%, more preferably by at least 12.5%, more specifically by at least 15% compared to the cytosine content of the wild type sequence.

According to the present invention, it is thus particularly preferred to increase the cytosine content of the modified mRNA by the maximum amount possible, in particular in the coding region for the at least one peptide or polypeptide, compared to the wild type sequence.

Preferably, the nucleosides, which are introduced into the modified mRNA in order to increase the G and/or C content, are selected from the group consisting of adenosine, cytosine, guanosine and uridine. Preferably, the modified RNA is modified only with respect to its G and/or C content as described herein, wherein (non-modified) cytosine or guanosine replaces other nucleosides in order to increase the G and/or C content as described herein. Thus, no analogs of the nucleosides adenosine, cytosine, guanosine and uridine, preferably no nucleoside or ribonucleoside analogs as defined herein, are preferably used.

Preferably, the modified mRNA is not modified with a chemical modification at the 4-, 5- or 6-position of the pyrimidine base of the nucleosides of cytidine and/or uridine; a chemical modification at the 2-, 6-, 7- or 8-position of the purine base of the nucleosides of adenosine, inosine and/or guanosine; and/or a chemical modification at the 2'-position of the sugar of the nucleosides of adenosine, inosine, guanosine, cytidine and/or uridine.

More preferably, the modified mRNA is not modified with a chemical modification at the 2-, 6-, 7- or 8-position of the purine base of the nucleosides of adenosine, inosine and/or guanosine; and a chemical modification at the 2'-position of the sugar of the nucleosides of adenosine, inosine, guanosine, cytidine and/or uridine.

Even more preferably, the modified mRNA is not modified with a chemical modification at the 4-, 5-or 6-position of the pyrimidine base of the nucleosides of cytidine and/or uridine; and a chemical modification at the 2'-position of the sugar of the nucleosides of adenosine, inosine, guanosine, cytidine and/or uridine.

Preferably, the modified mRNA is not modified with a chemical modification at the 5- or 6-position of the pyrimidine base of the nucleosides cytidine and/or uridine, wherein the chemical modification is preferably selected from the group consisting of 4-thio, 5-iodo-/ (5-I-), 5-bromo- / (5-Br-), 5-aminoallyl-, 5-fluoro- / (5-F-), 5-hydroxy-, 5-hydro- / (5-H-), 5-nitro-, 5-propynyl- / (5-(C C-CH3)-), 5-methyl-, 5-methyl-2-thio-, 5-formyl-, 5-hydroxymethyl-, 5-methoxy-, 5-oxyacetic acid methyl ester-, 5-oxyacetic acid-, 5-carboxyhydroxymethyl-, 5-(carboxyhydroxymethyl)pyrimidine methyl ester-, 5-methoxycarbonylmethyl-, 5-methoxycarbonylmethyl-2-thio, 5-aminomethyl-, 5-aminomethyl-2-thio-, 5-aminomethyl-2-seleno-, 5-methylaminomethyl-, 5-carbamoylmethyl-, 5-carboxymethylaminomethyl-, 5-carboxymethylaminomethyl-2-thio-, 5-carboxymethyl-, 5-methyldihydro-, 5-taurinomethyl-, 5-taurinomethyl-2-thiouridine, 5-isopentenylaminomethyl-, 5-isopentenylaminomethyl-2-thio-, 5-aminopropyl-/(5-(C3H6NH3)-),5-methoxy-ethoxy-methyl-/(5-(CH2-O-C2H4-O-CH3)-) and 6-aza-.

Further, the modified mRNA is not modified with a chemical modification at the 2-, 6-, 7- or 8-position of the purine base of the nucleosides adenosine, inosine and/or guanosine, wherein the chemical modification is preferably selected from the group consisting of 2-Amino-, 7-Deaza-, 8-Aza- and 8-Azido-.

In addition or alternatively, the modified mRNA is not modified with a chemical modification at the 2'-position of the sugar of the nucleosides adenosine, inosine, guanosine, cytidine and/or uridine, when incorporated in the RNA sequence, wherein such chemical modifications at the 2'-position of the sugar of the nucleosides adenosine, inosine, guanosine, cytidine and/or uridine may be selected from the group consisting of 2'-deoxy-, 2'-amino-2'-deoxy- / 2'-amino-, 2'-fluoro-2'-deoxy- / 2'-fluoro- and 2'-O-methyl-2'-deoxy- / 2'-O-methyl-.

Preferably, the modified mRNA is not modified with a chemical modification at the 4,- 5- or 6-position of the base pyrimidine of the nucleosides cytidine and/or uridine and at the 2'-position of the ribose sugar as defined above, wherein the chemical modification is preferably selected from the group consisting of 4-thio-2'-deoxy-, 4-thio-2'-amino-, 4-thio-2'-fluoro-, 4-thio-2'-O-methyl-, 5-iodo-2'-deoxy-, 5-iodo-2'-amino-, 5-iodo-2'-fluoro-, 5-iodo-2'-O-methyl-, 5-bromo-2'-deoxy-, 5-bromo-2'-amino-, 5-bromo-2'-fluoro-, 5-bromo-2'-O-methyl-, 5-aminoallyl-2'-deoxy-, 5-aminoallyl-2'-amino-, 5-aminoallyl-2'-fluoro-, 5-aminoallyl-2'-O-methyl-, 5-fluoro-2'-deoxy-, 5-fluoro-2'-amino-, 5-fluoro-2'-fluoro-, 5-fluoro-2'-O-methyl-, 5-hydroxy-2'-deoxy-, 5-hydroxy-2'-amino-, 5-hydroxy-2'-fluoro-, 5-hydroxy-2'-O-methyl-, 5-hydro-2'-deoxy-, 5-hydro-2'-amino-, 5-hydro-2'-fluoro-, 5-hydro-2'-O-methyl-, 5-nitro-2'-deoxy-, 5-nitro-2'-amino-, 5-nitro-2'-fluoro-, 5-nitro-2'-O-methyl-, 5-propynyl-2'-deoxy-, 5-propynyl-2'-amino-, 5-propynyl-2'-fluoro-, 5-propynyl-2'-O-methyl-, 5-methyl-2'-deoxy-, 5-methyl-2'-amino-, 5-methyl-2'-fluoro-, 5-methyl-2'-O-methyl (5,2'-O-dimethyl)-, 5-methyl-2-thio-2'-deoxy-, 5-methyl-2-thio-2'-amino-, 5-methyl-2-thio-2'-fluoro-, 5-methyl-2-thio-2'-O-methyl-, 5-formyl-2'-deoxy-, 5-formyl-2'-amino-, 5-formyl-2'-fluoro-, 5-formyl-2'-O-methyl-, 5-hydroxymethyl-2'-deoxy-, 5-hydroxymethyl-2'-amino-, 5-hydroxymethyl-2'-fluoro-, 5-hydroxymethyl-2'-O-methyl-, 5-methoxy-2'-deoxy-, 5-methoxy-2'-amino-, 5-methoxy-2'-fluoro-, 5-methoxy-2'-O-methyl-, 5-oxyacetic acid methyl ester-2'-deoxy-, 5-oxyacetic acid methyl ester-2'-amino-, 5-oxyacetic acid methyl ester-2'-fluoro-, 5-oxyacetic acid methyl ester-2'-O-methyl-, 5-oxyacetic acid-2'-deoxy-, 5-oxyacetic acid-2'-amino-, 5-oxyacetic acid-2'-fluoro-, 5-oxyacetic acid-2'-O-methyl-, 5-carboxyhydroxymethyl-2'-deoxy-, 5-carboxyhydroxymethyl-2'-amino-, 5-carboxyhydroxymethyl-2'-fluoro-, 5-carboxyhydroxymethyl-2'-O-methyl-, 5-(carboxyhydroxymethyl)pyrimidine methyl ester-2'-deoxy-, 5-(carboxyhydroxymethyl)pyrimidine methyl ester-2'-amino-, 5-(carboxyhydroxymethyl)pyrimidine methyl ester-2'-fluoro-, 5-(carboxyhydroxymethyl)pyrimidine methyl ester-2'-O-methyl-, 5-methoxycarbonylmethyl-2'-deoxy-, 5-methoxycarbonylmethyl-2'-amino-, 5-methoxycarbonylmethyl-2'-fluoro-, 5-methoxycarbonylmethyl-2'-O-methyl-, 5-methoxycarbonylmethyl-2-thio 2'-deoxy-, 5-methoxycarbonylmethyl-2-thio 2'-amino-, 5-methoxycarbonylmethyl-2-thio 2'-fluoro-, 5-methoxycarbonylmethyl-2-thio 2'-O-methyl-, 5-aminomethyl-2'-deoxy-, 5-aminomethyl-2'-amino-, 5-aminomethyl-2'-fluoro-, 5-aminomethyl-2'-O-methyl-, 5-aminomethyl-2-thio-2'-deoxy-, 5-aminomethyl-2-thio-2'-amino-, 5-aminomethyl-2-thio-2'-fluoro-, 5-aminomethyl-2-thio-2'-O-methyl-, 5-aminomethyl-2-seleno-2'-deoxy-, 5-aminomethyl-2-seleno-2'-amino-, 5-aminomethyl-2-seleno-2'-fluoro-, 5-aminomethyl-2-seleno-2'-O-methyl-, 5-methylaminomethyl-2'-deoxy-, 5-methylaminomethyl-2'-amino-, 5-methylaminomethyl-2'-fluoro-, 5-methylaminomethyl-2'-O-methyl-, 5-carbamoylmethyl-2'-deoxy-, 5-carbamoylmethyl-2'-amino-, 5-carbamoylmethyl-2'-fluoro-, 5-carbamoylmethyl-2'-O-methyl-, 5-carboxymethylaminomethyl-2'-deoxy-, 5-carboxymethylaminomethyl-2'-amino-, 5-carboxymethylaminomethyl-2'-fluoro-, 5-carboxymethylaminomethyl-2'-O-methyl-, 5-carboxymethylaminomethyl-2-thio-2'-deoxy-, 5-carboxymethylaminomethyl-2-thio-2'-amino-, 5-carboxymethylaminomethyl-2-thio-2'-fluoro-, 5-carboxymethylaminomethyl-2-thio-2'-O-methyl-, 5-carboxymethyl-2'-deoxy-, 5-carboxymethyl-2'-amino-, 5-carboxymethyl-2'-fluoro-, 5-carboxymethyl-2'-O-methyl-, 5-methyldihydro-2'-deoxy-, 5-methyldihydro-2'-amino-, 5-methyldihydro-2'-fluoro-, 5-methyldihydro-2'-O-methyl-, 5-taurinomethyl-2'-deoxy-, 5-taurinomethyl-2'-amino-, 5-taurinomethyl-2'-fluoro-, 5-taurinomethyl-2'-O-methyl-, 5-taurinomethyl-2-thiouridine-2'-deoxy-, 5-taurinomethyl-2-thiouridine-2'-amino-, 5-taurinomethyl-2-thiouridine-2'-fluoro-, 5-taurinomethyl-2-thiouridine-2'-O-methyl-, 5-isopentenylaminomethyl-2'-deoxy-, 5-isopentenylaminomethyl-2'-amino-, 5-isopentenylaminomethyl-2'-fluoro-, 5-isopentenylaminomethyl-2'-O-methyl-, 5-isopentenylaminomethyl-2-thio-2'-deoxy-, 5-isopentenylaminomethyl-2-thio-2'-amino-, 5-isopentenylaminomethyl-2-thio-2'-fluoro-, 5-isopentenylaminomethyl-2-thio-2'-O-methyl-, 5-aminopropyl-2'-deoxy-, 5-aminopropyl-2'-amino-, 5-aminopropyl-2'-fluoro-, 5-aminopropyl-2'-O-methyl-, 5-methoxy-ethoxy-methyl-2'-deoxy-, 5-methoxy-ethoxy-methyl-2'-amino-, 5-methoxy-ethoxy-methyl-2'-fluoro-, 5-methoxy-ethoxy-methyl-2'-O-methyl-, 6-aza-2'-deoxy-, 6-aza-2'-amino-, 6-aza-2'-fluoro- and 6-aza-2'-O-methyl-.

More preferably, the modified mRNA is not modified with a chemical modification at the 2-, 6-, 7- or 8-position of the purine base of the nucleosides adenosine, inosine and/or guanosine and at the 2'-position of the ribose sugar as defined above, wherein the chemical modification is selected from the group consisting of 2-Amino-2'-deoxy-, 2-Amino-2'-amino-, 2-Amino-2'-fluoro-, 2-Amino-2'-O-methyl-, 7-Deaza-2'-deoxy-, 7-Deaza-2'-amino-, 7-Deaza-2'-fluoro-, 7-Deaza-2'-O-methyl-, 8-Aza-2'-deoxy-, 8-Aza-2'-amino-, 8-Aza-2'-fluoro-, 8-Aza-2'-O-methyl-, 8-Azido-2'-deoxy-, 8-Azido-2'-amino-, 8-Azido-2'-fluoro- and 8-Azido-2'-O-methyl-.

According to the invention, the term polypeptide or protein refers to a peptide having at least 20, and preferably more than 50 amino acids. It may refer to a monomeric or multimeric protein. A peptide is understood to contain typically less than 20 amino acids.

According to the first embodiment of the present invention, the term "biologically active peptide, polypeptide or protein" refers to a polypeptide or protein that has the capability of performing one or more biological functions or a set of activities normally attributed to the polypeptide in a biological context. The biological activity may e.g. be a receptor binding activity, e.g. as a ligand, a catalytic activity, a transporter activity or an activity induced in cell structure proteins or modifications of cellular proteins, such as e.g. phosphorylation. The biological activity of the peptide, polypeptide or protein encoded by the wild type or by the modified mRNA according to the invention can be determined by any method available in the art. For example, the biological activity of members of the interferon family of proteins can be determined by any of a number of methods including their interaction with interferon-specific antibodies, their ability to increase resistance to viral infection, or their ability to modulate the transcription of interferon-regulated gene targets.

In a preferred embodiment, the biologically active peptide, polypeptide or protein does not comprise the gene product of a reporter gene or a marker gene. In the context of the present invention, the mRNA does preferably not encode, for instance, luciferase; green fluorescent protein (GFP) and its variants (such as eGFP, RFP or BFP); α-globin; hypoxanthine-guanine phosphoribosyltransferase (HGPRT); β-galactosidase; galactokinase; alkaline phosphatase; secreted embryonic alkaline phosphatase (SEAP)) or a resistance gene (such as a resistance gene against neomycin, puromycin, hygromycin and zeocin). In a preferred embodiment, the mRNA does not encode luciferase. In another embodiment, the mRNA does not encode GFP or a variant thereof.

In a further preferred embodiment, the biologically active peptide, polypeptide or protein does not comprise a protein (or a fragment of a protein) derived from a virus, preferably from a virus belonging to the family of Orthomyxoviridae. Preferably, the mRNA does not encode a protein that is derived from an influenza virus, more preferably an influenza A virus. Preferably, the mRNA does not encode an influenza A protein selected from the group consisting of hemagglutinin (HA), neuraminidase (NA), nucleoprotein (NP), matrix protein M1, matrix protein M2, NS1, NS2 (NEP: nuclear export protein), PA, PB1 (polymerase basic 1), PB1-F2 and PB2. In another preferred embodiment, the mRNA does not encode ovalbumin (OVA) or a fragment thereof. Preferably, the mRNA does not encode an influenza A protein or ovalbumin.

By the method according to the invention, an optimum balance of increased mRNA stability on the one hand and reduced immunostimulatory properties on the other hand is achieved. Increased C-content of the modified mRNA reduces the mRNA's immunogenicity and/or immunostimulatory capacity, while the increased C and G content contribute to increased mRNA stability. Increased mRNA stability means, e.g. increased *in vivo* functional stability resulting in an increased level of expressed protein over time. Accordingly, the *in vivo* AUC (area under the curve) of detectable protein upon mRNA administration is significantly increased for the inventive modified mRNA as compared to its wild type equivalent. Simultaneously, a reduced immune response in the patient is evoked (as determinable, for instance, by reduced amounts of cytokines secreted upon mRNA administration). The level of cytokine expression (secretion), e.g. TNF-a and IFN-α (e.g. by PBMCs) is reduced by at least 20%, preferably by at least 40%, as compared to the immune response triggered by the wild type equivalent. Such a reduction is measurable under *in vivo* and *in vitro* conditions.

According to the method of the present invention, the codon adaptation index of the modified target mRNA encoding at least one biologically active polypeptide or protein is increased by at least 0.05 - 0.5, or by at least 0.1 - 0.4, or by at least 0.2 - 0.3, or by at least 0.075 - 0.2, or by at least 0.1 - 0.2, or by at least 0.05, or by at least 0.1, or by at least 0.2, preferably by at least 0.125, more preferably by at least 0.15 compared to the CAI of the wild type mRNA.

The Codon Adaptation Index (CAI) is the most widespread technique for analyzing codon usage bias. The CAI provides an indication of gene expression level under the assumption that there is translational selection to optimize gene sequences according to their expression levels.

The codon adaptation index as used in the present invention can be calculated according to the method as published by Sharp and Li (Nucleic Acids Res. 1987 Feb 11;15(3):1281-95). Software-implemented solutions for the calculation of the CAI are also known in prior art, such as e.g. "The CAI Analyser Package" (Ramazotti et al, In Silico Biol. 2007; 7(4-5):507-26) and are thus readily available to the skilled person. CAI is simply defined as the geometric mean of the weight associated to each codon over the length of the gene sequence (measured in codons). For each amino acid, the weight of each of its codons, in CAI, is computed as the ratio between the observed frequency of the codon and the frequency of the synonymous codon for that amino acid. The CAI uses a reference set of highly expressed genes from a species to determine relative merit of each codon to calculate a score for a gene from the frequency of use of all codons in said gene. This index is useful for predicting the level of expression of a gene and indicate likely success of heterologous gene expression in a given cell system.

Within the scope of the present invention, the term codon adaptation index (CAI) as used herein relates to the codon usage frequency of the genes encoding the at least one polypeptide or protein. Codon adaptation is the adaptation of the codons of an open reading frame to the synonymous codons preferred in human / mammalian genes whilst avoiding the introduction of unwanted secondary sequence functions that impede expression of the resulting open reading frames.

Within the context of the present invention, a CAI score of 1 means that the optimal codon is used for each amino acid in each codon position. Thus, according to the methods of the present invention, the coding region of the mRNA has a CAI which is greater than the CAI of the corresponding wild coding type sequence and the CAI of the modified mRNA is sufficiently close to 1, at least 0.6, more preferably at least 0.7, more preferably at least 0.8 and more preferably at least 0.9, such that the desired level of expression of the at least one biologically active polypeptide or protein is achieved. Accordingly the CAI of the modified mRNA is at least greater by 0.05 - 0.5, or at least greater by 0.1 - 0.4, or at least greater by 0.2 - 0.3, or at least greater by 0.075 - 0.2, or at least greater by 0.1 - 0.2, or at least greater by 0.05, or at least greater by 0.1, or at least greater by 0.2, preferably at least greater by 0.125, more preferably at least greater by 0.15, than the CAI of the wild type mRNA region coding for the at least one biologically active polypeptide or protein.

According to a preferred embodiment, the present invention provides a modified mRNA encoding at least one biologically active polypeptide or protein, which is obtainable or obtained by at least one or more embodiments of the inventive method of the present invention and wherein the modified mRNA is characterized by a reduced immunogenicity and/or immunostimulatory capacity compared to the wild type mRNA and may also be characterized by enhanced stability, in particular enhanced functional stability, as compared to the wild type equivalent.

In a more preferred embodiment of the present invention, the obtainable mRNA may further be subject to a step of determining the immunogenicity and/or immunostimulatory capacity of the inventive modified mRNA. Such a step comprises the e.g. *in vitro* sub-steps of transfecting competent cells, e.g. PBMCs, with the modified mRNA according to one or more embodiments of the inventive method, cultivating the cells, e.g. for 8h - 24h, preferably for 12h - 48h, preferably for 18h - 24h, preferably for 24 - 48h, preferably for at least 12 hours, preferably for at least 18h, more preferably for at least 20h and determining the amount of pro-inflammatory cytokines in the cell supernatant. The amount of pro-inflammatory cytokines present in the supernatant of the cells transfected with the modified mRNA according to the invention is compared to the amount of pro-inflammatory cytokines present in the supernatant of cells transfected with the wild type mRNA equivalent.

Appropriate techniques for determining the immunogenicity and/or immunostimulatory capacity of a nucleic acid, such as that of e.g. the inventive modified mRNA, are known in the art and are readily available to the skilled person (Robbins et al., 2009. Oligonucleotides 19(2):89-102). The nature and the extent of the cytokine response to RNA depends on several factors including timing, cell type, delivery vehicle and route of administration. The absence of immunostimulation at a single time point for a single cytokine does not necessarily demonstrate the absence of immunostimulation in general, such that assessment of several cytokine responses at multiple time points may be required. Antibodies and ELISA kits for the determination of interferons (e.g. IFNa and IFNβ) and a variety of pro-inflammatory cytokines, such as e.g. TNFα, TGFβ, IL-1 and IL-6, are commercially available.

If it were desired to carry out *in vivo* studies for testing for IFNa and/or suitable pro-inflammatory cytokines, such as e.g. TNFα and IL-6, their presence in the plasma of treated animals can be used to monitor the systemic activation of the immune response. Measurement of the immune response at an appropriate time point after mRNA administration is critical for a valid assessment. Systemic administration of mRNA formulations to mice leads to detectable elevations of serum cytokines within 1 to 2 hours, depending on the type of delivery vehicle and the cytokine of interest. Typically, the increase of cytokine levels in the serum is transient and may decrease again after 12 to 24 hours of treatment. For example, mice can be injected with complexed mRNA and serum levels of, e.g., IFNa, TNFα and IL-6 may be measured 6 hours post injection by using suitable ELISA assays (Kariko et al., 2012. Mol. Ther. 20(5):948-53).

For *in vitro* studies, a similar panel of cytokines can be used to assess the stimulation of primary immune cell cultures after treatment with mRNA of the invention. The secretion of cytokines is cell type-dependent and therefore several cytokines may be required to be tested. The assessment of cytokine responses from *in vitro* cell cultures is less time critical compared to *in vivo* studies because the secreted cytokines tend to accumulate in the cell culture supernatant. Therefore, the measurement of cytokines at a single time point, such as between 8h - 24h, or between 16 - 24 h, or between 12h - 48h, or between 18h - 24h, or between 24 - 48h, preferably 12 hours, preferably 18h, more preferably 20h, after mRNA administration, such as e.g. the treatment with the modified mRNA according to the present invention is often sufficient to detect an immune response. For example, a human *in vitro* whole blood assay (WBA) or peripheral blood mononuclear cell (PBMC) based assay can be used to assess the cytokine response elicited by treatment with modified mRNA molecules of the invention (Coch et al., 2013. PLoS One 8(8):e71057), as exemplified in the appended examples, in particular Example 2. Alternatively, primary myeloid and plasmacytoid dendritic cells (pDCs) can be isolated from peripheral blood using commercially available cell isolation kits and are treated with mRNA of the invention, such as the modified mRNA according to the invention, in cell culture. After 8 to 20 hours of incubation cytokine levels (e.g. IFNα, TNFα and/or IL-8) in the cell culture medium may be determined with ELISA assays. In addition, the activation status of DCs may be analyzed by measuring cell surface expression of activation markers (e.g. CD80, CD83, CD86 and MHC class II) by flow cytometry (Kariko et al., 2005. Immunity 23(2):165-75).

Alternatively, activation of the cytosolic RNA sensor RIG-I in cells can be assessed by treating cells with modified mRNA of the invention, generating cell lysates, separating proteins by SDS-polyacrylamide gel electrophoresis and probing for phosphorylation of PKR by Western blotting. Phosphorylation of PKR on threonine at position 446 (pT446) is required for PKR activation and thus represents an activation marker. In addition, the phosphorylation of the alpha subunit of eukaryotic protein synthesis initiation factor-2 (EIF2-alpha), which is a physiological substrate for PKR, can be assessed with an antibody directed at phosphorylated serine at position 51 (pS51) (Anderson et al., 2010. Nucleic Acids Res. 38(17):5884-92).

The activation of 2'-5' oligoadenylate synthetase (OAS) by RNA can be tested in an enzyme assay. The assay for OAS can be performed by the measurement of the 2-5(A) products. Using [³H]ATP in the synthetase reaction, the conversion of 2-5(A) is measured by digestion of the reaction products with alkaline phosphatase. This converts [³H]2-5(A) to core (A2'p)ₙA, which is negatively charged and binds to DEAE-cellulose paper. Residual [³H]ATP is converted to adenosine which is uncharged and removed by washing. This assay is suitable to measure OAS activity in crude cell extracts as well as that of purified OAS (Sharp et al., 1999. Virology 257(2):303-13; Anderson et al., 2011. Nucleic Acids Res. 39(21):9329-38).

Typically, the inventive modified mRNA obtainable according to one or more embodiments of the inventive method for targeted modulation of the immune response against an mRNA is characterized by lower affinity to one or more of TLR3, TLR7, TLR8, MDA5, RIG-I, LGP2 or 2'-5'-oligoadenylate synthetase compared to the wild type mRNA encoding the same peptide, polypeptide or protein.

The term "affinity" as used herein relates to the ability of an RNA, such as e.g. the modified mRNA of the invention, to bind to and/or to function as a ligand and/or activate signaling events by one or more of TLR3, TLR7, TLR8, MDA5, RIG-I, LGP2 or 2'-5'-oligoadenylate synthetase. Thus, RNA, such as the modified mRNA of the present invention, which is characterized by lower affinity to one or more of TLR3, TLR7, TLR8, MDA5, RIG-I, LGP2 or 2'-5'-oligoadenylate synthetase will result in a reduced production of pro-inflammatory cytokines, such as, e.g. TNFα, TGFβ, IL-1 or IL-6. The affinity may be reduced by at least 50%, preferably by at least 60%, as compared to the affinity of the wild type equivalent.

Thus, the affinity of the modified mRNA of the present invention to one or more of TLR3, TLR7, TLR8, MDA5, RIG-I, LGP2 or 2'-5'-oligoadenylate synthetase is correlated with the amount of pro-inflammatory cytokines produced in any one of the above assays, i.e. a reduced affinity and/or activation of one or more of TLR3, TLR7, TLR8, MDA5, RIG-I, LGP2 or 2'-5'-oligoadenylate synthetase is correlated with a reduced amount of pro-inflammatory cytokines released into the cell supernatant compared to the amount of pro-inflammatory cytokines, such as, e.g. TNFα, TGFβ, IL-1 or IL-6, produced in response to transfection of corresponding wild type mRNA encoding the at least one biologically active polypeptide or protein.

In a preferred embodiment, the inventive method of targeted modulation of the immune response against an mRNA encoding at least one biologically active polypeptide or protein according to the present invention may be executed on a computer with the aid of suitable software by way of executing at least one algorithm thereon.

Computer software for sequence analysis and/or codon optimization is known in prior art and is readily accessible to the skilled person. Computer programs, which may be used to execute the at least one algorithm of the inventive method may include, e.g. CodonW, GCUA, INCA, etc. Also, several software packages that are available online may be used, such as e.g. the "JCat" available at http://www.jcat.de/. The at least one algorithm may, e.g. comprise the following steps:
1. Providing the wild type target mRNA sequence, such as e.g. the entire mRNA or a portion thereof. Go to step 2.
2. Generate a novel modified mRNA sequence by modifying the target sequence according to the method as provided in any one of claims 1 - 11. Go to step 3.
3. Evaluate the modified mRNA sequence and determine if it has a predetermined property, e.g. at least 70% of the codons of the wild type mRNA sequence, which are cytosine content optimizable, have been modified to the maximum C-content possible without altering the amino acid sequence compared to the wild type sequence. If the modified mRNA sequence has the predetermined property, then proceed to step 4, otherwise proceed to step 2.
4. Providing the modified mRNA sequence as an optimized and modified mRNA.

The modified mRNA sequence obtained by the above algorithm may then be used as a template for e.g. chemical RNA synthesis or in vitro transcription.

According to a further embodiment the inventive modified mRNA coding for at least one biologically active peptide, polypeptide or protein may be obtained by synthesis, such as e.g. chemical synthesis.

Defined chemical synthesis of the modified mRNA of the invention in the 3' -> 5' direction is well established in prior art. The technology utilizes a ribonucleoside with suitable N- protecting group: generally 5'- Protecting group, the most popular being dimethoxytriphenyl, i.e. the DMT group; T- protecting group, out of which most popular is t-Butyldimethylsilyl ether; and, a 3'- phosphoramidite, the most popular of which is cyanoethyl diisopropyl (component 1). This component is then coupled with a nucleoside with a suitable N- protecting group, 2' or 3' succinate of a ribonucleoside attached to a solid support (component 2). The coupling of component 1 and 5'-OH-n-protected -2',3'-protected-nucleoside (component 3) are also achieved in solution phase in presence of an activator leading to dimers and oligoribonucleotides, followed by oxidation (3'->5' direction synthesis), also leads to a protected dinucleotide having a 3'-5'- internucleotide linkage (Ogilvie, K.K., Can. J. Chem., 58, 2686, 1980). Other technologies for chemical RNA synthesis, i.e. for the synthesis of the modified RNA according to the invention are known in prior art, such as, e.g. the method disclosed in US 20110275793 A1.

According to a more preferred embodiment, the modified mRNA according to the inventive method of the present invention may be obtained by *in vitro* transcription, preferably by bacteriophage-mediated *in vitro* transcription, preferably by Sp6 polymerase *in vitro* transcription and/or T3 polymerase-mediated *in vitro* transcription, more preferably by T7 polymerase-mediated *in vitro* transcription.

Highly efficient *in vitro* transcription systems have been developed in prior art, particularly ones using phage polymerases such as T7, SP6, and T3. The DNA-dependent phage T7, T3, and SP6 RNA polymerases are widely used to synthesize a large quantity of RNAs. These enzymes are highly processive and are thus capable of generating long RNA molecules of up to thousands of nucleotides in length with low probability of falling off DNA templates during transcription and may thus be used for *in* vitro transcription in the present invention. Phage RNA polymerases specifically recognize their 18-bp promoter sequences (T7, 5'-TAATACGACTCACTATAG; T3, 5'-AATTAACCCTCACTAAAG; and SP6, 5'-ATTTAGGTGACACTATAG) and initiate transcription precisely at the 18th nucleotide guanosine. With a T7, T3, or SP6 promoter fused to the 5' end of a DNA template, the transcription reaction is expected to generate an RNA molecule with the predicted sequence.

In this method, a DNA molecule corresponding to the modified mRNA of the present invention is transcribed *in vitro* for the production of the mRNA. This DNA matrix has a suitable promoter, for example a T7 and/or SP6 and/or T3 promoter, for the *in vitro* transcription, followed by the desired nucleotide sequence for the mRNA to be produced and a termination signal for the *in vitro* transcription. According to the invention the DNA molecule that forms the matrix of the RNA construct to be produced, such as e.g. the modified mRNA according to the present invention is prepared by fermentative replication and subsequent isolation as part of a plasmid replicable in bacteria.

Suitable plasmids for *in vitro* transcription of the modified mRNA according to the present invention are known in the art and are commercially available. For example the following plasmids may be mentioned as examples pT7Ts (GeneBank Accession No. U26404), the pGEM® series, for example pGEM®-1 (GeneBank Accession No. X65300) and pSP64 (GeneBank-Accession No. X65327); see also Mezei and Storts, Purification of PCR Products, in: Griffin and Griffin (Eds.), PCR Technology: Current Innovation, CRC Press, Boca Raton, FL, 2001. The *in vitro* transcription of the modified mRNA according to the present invention may also include ribonucleoside triphosphates (rNTPs) analogues, such as those, e.g. required for 5' capping of the *in vitro* transcribed modified mRNA according to the invention. rNTP analogues other than those naturally present in mRNAs, in particular mammalian mRNAs, such as e.g. human mRNAs, should not be used for *in vitro* transcription, since the mRNA of the inventive mRNA may be disadvantageously affected and, more importantly, if the *in vitro* transcribed inventive mRNA is to be used in protein replacement therapy, this may not be in accordance with national regulatory affairs.

According to a more preferred embodiment, the modified mRNA that is obtainable by the method according to the present invention may be synthesized by *in vitro* transcription including naturally occurring rNTP analogues, for example 5-methyl-cytidine triphosphate and/or pseudouridine triphosphate.

According to a further preferred embodiment, the modified mRNA obtainable by the inventive method may be synthesized by *in vitro* transcription, including other than naturally occurring rNTP analogues.

The term "ribonucleoside triphosphate analogues" as used herein refers to ribonucleoside triphosphate compounds comprising a chemical modification, wherein the chemical modification may comprise a backbone modification, a sugar modification, or a base modification. These ribonucleoside triphosphate analogues are also termed herein as modified nucleoside triphosphates, modified ribonucleosides or modified nucleosides.

In this context, the modified nucleoside triphosphates as defined herein are nucleotide analogs/modifications, e.g. backbone modifications, sugar modifications or base modifications. A backbone modification in the context of the present invention is a modification, in which phosphates of the backbone of the nucleotides are chemically modified. A sugar modification in the context of the present invention is a chemical modification of the sugar of the nucleotides. Furthermore, a base modification in in the context of the present invention is a chemical modification of the base moiety of the nucleotides. In this context nucleotide analogs or modifications are preferably selected from nucleotide analogs, which are applicable for transcription and/or translation.

### Sugar Modifications

The modified nucleosides and nucleotides, which may be used in the context of the present invention, can be modified in the sugar moiety. For example, the 2' hydroxyl group (OH) can be modified or replaced with a number of different "oxy" or "deoxy" substituents. Examples of "oxy" -2' hydroxyl group modifications include, but are not limited to, alkoxy or aryloxy (-OR, e.g., R = H, alkyl, cycloalkyl, aryl, aralkyl, heteroaryl or sugar); polyethyleneglycols (PEG), -0(CH2CH2o)nCH2CH2OR; "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH2; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration with respect to that of the corresponding carbon in ribose. Thus, a modified nucleotide can include nucleotides containing, for instance, arabinose as the sugar.

### Backbone Modifications

The phosphate backbone may further be modified in the modified nucleosides and nucleotides. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

### Base Modifications

The modified nucleosides and nucleotides, which may be used in the present invention, can further be modified in the nucleobase moiety. Examples of nucleobases found in RNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group.

In particularly preferred embodiments of the present invention, the nucleotide analogs/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-lodo-2'-deoxycytidine-5'-triphosphate, 5-iodouridine-5'-triphosphate, 5-lodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, I-taurinomethyl-4-thio-uridine, 5-methyl-uridine, 1-methyl-pseudouridine, 4-thio-1-methyl-pseudouridine, 2-thio-l-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1 - methyl- 1 -deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-I-methyl-pseudoisocytidine .

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, I-methyl-6-thio-guanosine, N2-methyl-6-thio-guanosine, and N2,N2-dimethyl-6-thio-guanosine.

In some embodiments, the nucleotide can be modified on the major groove face and can include replacing hydrogen on C-5 of uracil with a methyl group or a halo group.
In specific embodiments, a modified nucleoside is 5'-0-(I-Thiophosphate)-Adenosine, 5 ' -0-(1-Thiophosphate)-Cytidine, 5'-0-(1-Thiophosphate)-Guanosine, 5'-0-(1-Thiophosphate)-Uridine or 5'-0-(I-Thiophosphate)-Pseudouridine.

In further specific embodiments the modified nucleotides include nucleoside modifications selected from 6-aza-cytidine, 2-thio-cytidine, -thio-cytidine, Pseudo-iso-cytidine, 5-aminoallyl-uridine, 5-iodo-uridine, N1-methyl-pseudouridine, 5,6-dihydrouridine, -thio-uridine, 4-thio-uridine, 6-aza-uridine, 5-hydroxy-uridine, deoxythymidine, 5-methyl-uridine, Pyrrolo-cytidine, inosine, -thio-guanosine, 6-methyl-guanosine, 5-methyl-cytdine, 8-oxo-guanosine, 7-deaza-guanosine, N1-methyladenosine, 2-amino-6-Chloro-purine, N6-methyl-2-amino-purine, Pseudo-iso-cytidine, 6-Chloro-purine, N6-methyl-adenosine, -thio-adenosine, 8-azido-adenosine, 7-deaza-adenosine.

Further modified nucleotides have been described previously (WO2013052523).

In some embodiments of the present invention, the modified mRNA obtainable by the method according to the invention does not comprise any modified nucleosides as described above. In these embodiments, the modified mRNA preferably comprises exclusively nucleosides, which are selected from the group consisting of adenosine, cytosine, guanosine and uridine. Preferably, the modified mRNA is modified only with respect to its G and/or C content as described herein, wherein (non-modified) cytosine or guanosine replaces other nucleosides in order to increase the G and/or C content as described herein.

Preferably, the modified mRNA that is obtainable by the method according to the invention has not been modified with a chemical modification at the 4-, 5- or 6-position of the pyrimidine base of the nucleosides of cytidine and/or uridine; a chemical modification at the 2-, 6-, 7- or 8-position of the purine base of the nucleosides of adenosine, inosine and/or guanosine; and/or a chemical modification at the 2'-position of the sugar of the nucleosides of adenosine, inosine, guanosine, cytidine and/or uridine.

More preferably, the modified mRNA that is obtainable by the method according to the invention has not been modified with a chemical modification at the 2-, 6-, 7- or 8-position of the purine base of the nucleosides of adenosine, inosine and/or guanosine; and a chemical modification at the 2'-position of the sugar of the nucleosides of adenosine, inosine, guanosine, cytidine and/or uridine.

Even more preferably, the modified mRNA that is obtainable by the method according to the invention has not been modified with a chemical modification at the 4-, 5-or 6-position of the pyrimidine base of the nucleosides of cytidine and/or uridine; and a chemical modification at the 2'-position of the sugar of the nucleosides of adenosine, inosine, guanosine, cytidine and/or uridine.

Preferably, the modified mRNA that is obtainable by the method according to the invention has not been modified with a chemical modification at the 5- or 6-position of the pyrimidine base of the nucleosides cytidine and/or uridine, wherein the chemical modification is preferably selected from the group consisting of 4-thio, 5-iodo- / (5-I-), 5-bromo- / (5-Br-), 5-aminoallyl-, 5-fluoro- / (5-F-), 5-hydroxy-, 5-hydro- / (5-H-), 5-nitro-, 5-propynyl- / (5-(C C-CH3)-), 5-methyl-, 5-methyl-2-thio-, 5-formyl-, 5-hydroxymethyl-, 5-methoxy-, 5-oxyacetic acid methyl ester-, 5-oxyacetic acid-, 5-carboxyhydroxymethyl-, 5-(carboxyhydroxymethyl)pyrimidine methyl ester-, 5-methoxycarbonylmethyl-, 5-methoxycarbonylmethyl-2-thio, 5-aminomethyl-, 5-aminomethyl-2-thio-, 5-aminomethyl-2-seleno-, 5-methylaminomethyl-, 5-carbamoylmethyl-, 5-carboxymethylaminomethyl-, 5-carboxymethylaminomethyl-2-thio-, 5-carboxymethyl-, 5-methyldihydro-, 5-taurinomethyl-, 5-taurinomethyl-2-thiouridine, 5-isopentenylaminomethyl-, 5-isopentenylaminomethyl-2-thio-, 5-aminopropyl- / (5-(C3H6NH3)-), 5-methoxy-ethoxy-methyl-/ (5-(CH2-O-C2H4-O-CH3)-) and 6-aza-.

Further, the modified mRNA that is obtainable by the method according to the invention has preferably not been modified with a chemical modification at the 2-, 6-, 7- or 8-position of the purine base of the nucleosides adenosine, inosine and/or guanosine, wherein the chemical modification is preferably selected from the group consisting of 2-Amino-, 7-Deaza-, 8-Aza- and 8-Azido-.

In addition or alternatively, the modified mRNA that is obtainable by the method according to the invention has preferably not been modified with a chemical modification at the 2'-position of the sugar of the nucleosides adenosine, inosine, guanosine, cytidine and/or uridine, when incorporated in the RNA sequence, wherein such chemical modifications at the 2'-position of the sugar of the nucleosides adenosine, inosine, guanosine, cytidine and/or uridine may be selected from the group consisting of 2'-deoxy-, 2'-amino-2'-deoxy- / 2'-amino-, 2'-fluoro-2'-deoxy- / 2'-fluoro- and 2'-O-methyl-2'-deoxy- / 2'-O-methyl-.

Preferably, the modified mRNA that is obtainable by the method according to the invention has not been modified with a chemical modification at the 4,- 5- or 6-position of the base pyrimidine of the nucleosides cytidine and/or uridine and at the 2'-position of the ribose sugar as defined above, wherein the chemical modification is preferably selected from the group consisting of 4-thio-2'-deoxy-, 4-thio-2'-amino-, 4-thio-2'-fluoro-, 4-thio-2'-O-methyl-, 5-iodo-2'-deoxy-, 5-iodo-2'-amino-, 5-iodo-2'-fluoro-, 5-iodo-2'-O-methyl-, 5-bromo-2'-deoxy-, 5-bromo-2'-amino-, 5-bromo-2'-fluoro-, 5-bromo-2'-O-methyl-, 5-aminoallyl-2'-deoxy-, 5-aminoallyl-2'-amino-, 5-aminoallyl-2'-fluoro-, 5-aminoallyl-2'-O-methyl-, 5-fluoro-2'-deoxy-, 5-fluoro-2'-amino-, 5-fluoro-2'-fluoro-, 5-fluoro-2'-O-methyl-, 5-hydroxy-2'-deoxy-, 5-hydroxy-2'-amino-, 5-hydroxy-2'-fluoro-, 5-hydroxy-2'-O-methyl-, 5-hydro-2'-deoxy-, 5-hydro-2'-amino-, 5-hydro-2'-fluoro-, 5-hydro-2'-O-methyl-, 5-nitro-2'-deoxy-, 5-nitro-2'-amino-, 5-nitro-2'-fluoro-, 5-nitro-2'-O-methyl-, 5-propynyl-2'-deoxy-, 5-propynyl-2'-amino-, 5-propynyl-2'-fluoro-, 5-propynyl-2'-O-methyl-, 5-methyl-2'-deoxy-, 5-methyl-2'-amino-, 5-methyl-2'-fluoro-, 5-methyl-2'-O-methyl (5,2'-O-dimethyl)-, 5-methyl-2-thio-2'-deoxy-, 5-methyl-2-thio-2'-amino-, 5-methyl-2-thio-2'-fluoro-, 5-methyl-2-thio-2'-O-methyl-, 5-formyl-2'-deoxy-, 5-formyl-2'-amino-, 5-formyl-2'-fluoro-, 5-formyl-2'-O-methyl-, 5-hydroxymethyl-2'-deoxy-, 5-hydroxymethyl-2'-amino-, 5-hydroxymethyl-2'-fluoro-, 5-hydroxymethyl-2'-O-methyl-, 5-methoxy-2'-deoxy-, 5-methoxy-2'-amino-, 5-methoxy-2'-fluoro-, 5-methoxy-2'-O-methyl-, 5-oxyacetic acid methyl ester-2'-deoxy-, 5-oxyacetic acid methyl ester-2'-amino-, 5-oxyacetic acid methyl ester-2'-fluoro-, 5-oxyacetic acid methyl ester-2'-O-methyl-, 5-oxyacetic acid-2'-deoxy-, 5-oxyacetic acid-2'-amino-, 5-oxyacetic acid-2'-fluoro-, 5-oxyacetic acid-2'-O-methyl-, 5-carboxyhydroxymethyl-2'-deoxy-, 5-carboxyhydroxymethyl-2'-amino-, 5-carboxyhydroxymethyl-2'-fluoro-, 5-carboxyhydroxymethyl-2'-O-methyl-, 5-(carboxyhydroxymethyl)pyrimidine methyl ester-2'-deoxy-, 5-(carboxyhydroxymethyl)pyrimidine methyl ester-2'-amino-, 5-(carboxyhydroxymethyl)pyrimidine methyl ester-2'-fluoro-, 5-(carboxyhydroxymethyl)pyrimidine methyl ester-2'-O-methyl-, 5-methoxycarbonylmethyl-2'-deoxy-, 5-methoxycarbonylmethyl-2'-amino-, 5-methoxycarbonylmethyl-2'-fluoro-, 5-methoxycarbonylmethyl-2'-O-methyl-, 5-methoxycarbonylmethyl-2-thio 2'-deoxy-, 5-methoxycarbonylmethyl-2-thio 2'-amino-, 5-methoxycarbonylmethyl-2-thio 2'-fluoro-, 5-methoxycarbonylmethyl-2-thio 2'-O-methyl-, 5-aminomethyl-2'-deoxy-, 5-aminomethyl-2'-amino-, 5-aminomethyl-2'-fluoro-, 5-aminomethyl-2'-O-methyl-, 5-aminomethyl-2-thio-2'-deoxy-, 5-aminomethyl-2-thio-2'-amino-, 5-aminomethyl-2-thio-2'-fluoro-, 5-aminomethyl-2-thio-2'-O-methyl-, 5-aminomethyl-2-seleno-2'-deoxy-, 5-aminomethyl-2-seleno-2'-amino-, 5-aminomethyl-2-seleno-2'-fluoro-, 5-aminomethyl-2-seleno-2'-O-methyl-, 5-methylaminomethyl-2'-deoxy-, 5-methylaminomethyl-2'-amino-, 5-methylaminomethyl-2'-fluoro-, 5-methylaminomethyl-2'-O-methyl-, 5-carbamoylmethyl-2'-deoxy-, 5-carbamoylmethyl-2'-amino-, 5-carbamoylmethyl-2'-fluoro-, 5-carbamoylmethyl-2'-O-methyl-, 5-carboxymethylaminomethyl-2'-deoxy-, 5-carboxymethylaminomethyl-2'-amino-, 5-carboxymethylaminomethyl-2'-fluoro-, 5-carboxymethylaminomethyl-2'-O-methyl-, 5-carboxymethylaminomethyl-2-thio-2'-deoxy-, 5-carboxymethylaminomethyl-2-thio-2'-amino-, 5-carboxymethylaminomethyl-2-thio-2'-fluoro-, 5-carboxymethylaminomethyl-2-thio-2'-O-methyl-, 5-carboxymethyl-2'-deoxy-, 5-carboxymethyl-2'-amino-, 5-carboxymethyl-2'-fluoro-, 5-carboxymethyl-2'-O-methyl-, 5-methyldihydro-2'-deoxy-, 5-methyldihydro-2'-amino-, 5-methyldihydro-2'-fluoro-, 5-methyldihydro-2'-O-methyl-, 5-taurinomethyl-2'-deoxy-, 5-taurinomethyl-2'-amino-, 5-taurinomethyl-2'-fluoro-, 5-taurinomethyl-2'-O-methyl-, 5-taurinomethyl-2-thiouridine-2'-deoxy-, 5-taurinomethyl-2-thiouridine-2'-amino-, 5-taurinomethyl-2-thiouridine-2'-fluoro-, 5-taurinomethyl-2-thiouridine-2'-O-methyl-, 5-isopentenylaminomethyl-2'-deoxy-, 5-isopentenylaminomethyl-2'-amino-, 5-isopentenylaminomethyl-2'-fluoro-, 5-isopentenylaminomethyl-2'-O-methyl-, 5-isopentenylaminomethyl-2-thio-2'-deoxy-, 5-isopentenylaminomethyl-2-thio-2'-amino-, 5-isopentenylaminomethyl-2-thio-2'-fluoro-, 5-isopentenylaminomethyl-2-thio-2'-O-methyl-, 5-aminopropyl-2'-deoxy-, 5-aminopropyl-2'-amino-, 5-aminopropyl-2'-fluoro-, 5-aminopropyl-2'-O-methyl-, 5-methoxy-ethoxy-methyl-2'-deoxy-, 5-methoxy-ethoxy-methyl-2'-amino-, 5-methoxy-ethoxy-methyl-2'-fluoro-, 5-methoxy-ethoxy-methyl-2'-O-methyl-, 6-aza-2'-deoxy-, 6-aza-2'-amino-, 6-aza-2'-fluoro- and 6-aza-2'-O-methyl-.

More preferably, the modified mRNA that is obtainable by the method according to the invention has not been modified with a chemical modification at the 2-, 6-, 7- or 8-position of the purine base of the nucleosides adenosine, inosine and/or guanosine and at the 2'-position of the ribose sugar as defined above, wherein the chemical modification is selected from the group consisting of 2-Amino-2'-deoxy-, 2-Amino-2'-amino-, 2-Amino-2'-fluoro-, 2-Amino-2'-O-methyl-, 7-Deaza-2'-deoxy-, 7-Deaza-2'-amino-, 7-Deaza-2'-fluoro-, 7-Deaza-2'-O-methyl-, 8-Aza-2'-deoxy-, 8-Aza-2'-amino-, 8-Aza-2'-fluoro-, 8-Aza-2'-O-methyl-, 8-Azido-2'-deoxy-, 8-Azido-2'-amino-, 8-Azido-2'-fluoro- and 8-Azido-2'-O-methyl-.

According to a further embodiment, the modified mRNA, which is obtained according to one or more embodiments of the inventive method of targeted modulation of the immune response against an mRNA, is devoid of destabilizing sequence elements (DSE) in the 3' and/or 5' UTR.

As used herein, the term "destabilizing sequence element" (DSE) refers to a sequence of nucleotides, which reduces the half-life of a transcript, e.g. the half-life of the inventive modified mRNA inside a cell and/or organism, e.g. a human. Accordingly, a DSE comprises a sequence of nucleotides, which reduces the intracellular half-life of an RNA transcript.

DSE sequences are found in short-lived mRNAs such as, for example; c-fos, c-jun, c-myc, GM-CSF, IL-3, TNF -alpha, IL-2, IL-6, IL-8, IL-10, Urokinase, bcl-2, SGLT1 (Na(+)-coupled glucose transporter), Cox-2 (cyclooxygenase 2), IL8, PAI-2 (plasminogen activator inhibitor type 2), betal -adrenergic receptor, GAP43 (5 'UTR and 3 'UTR). AU-rich elements (AREs) and/or U-rich elements (UREs), including but not limited to single, tandem or multiple or overlapping copies of the nonamer UUAUUUA(U/A)(U/A) (where U/A is either an A or a U) and/or the pentamer AUUUA and/or the tetramer AUUU. Accordingly, the DNA template used for *in vitro* transcription of the modified mRNA according to the present invention are devoid of the corresponding sequences.

Assays for measuring RNA degradation, such as e.g. degradation of the modified mRNA of the invention and thus the stability of the inventive modified mRNA are known in the art. For example, RNA half-life can be measured by the methods described by Duan and Jefcoate (Duan and Jefcoate, 2007. J. Mol. Endocrinol. 38(1-2):159-79).

According to this method, cells may e.g. be plated in 12-well plates at 25% density 24 hours prior to transfection. For transfection 1µg of modified mRNA of the invention may be transfected per well. The following proportion may be used: 1 µg mRNA, 1 µl Translt-mRNA reagent, 1 µl mRNA boost reagent and 100 µl serum-free media. The modified mRNA of the invention may then first be mixed with serum-free media, then RNA boost reagent may be added and mixed, followed by the addition of transfection reagent. The mixture may then be incubated at room temperature for three minutes and aliqoted directly onto cells in, e.g. complete media. Twelve hours after transfection, when cell mRNA levels have reached a steady state, cells may be washed once and cell culture medium may be exchanged for complete medium devoid of the transfection reagents (mixture) (zero hour time point). The degradation of the modified mRNA of the invention within the cells may then be determined by harvesting cells at appropriate time points, such as e.g. 12h and/or 16h and/or 18h and/or 20h and/or 24 and/or 48h post transfection. Total cellular RNA may subsequently be isolated and the amount of modified mRNA of the invention may be determined by reverse transcription and/or quantitative real-time PCR using specific primers that bind to the coding region of the modified mRNA of the invention and/or the 5' and/or 3' UTRs of the modified mRNA. The half-life of the modified mRNA of the invention may then be calculated by linear regression fit of the time points on semi-log plots.

In a preferred embodiment the inventive modified mRNA may further comprise a 5'-CAP structure and/or a polyA-tail of at least 60 nucleotides, more preferably of at least 70 nucleotides and/or a 3' stabilizing sequence.

Within the present invention the term "CAP structure" refers to a structure found on the 5'-end of an mRNA, such as e.g. the modified mRNA according to one or more embodiments of the method of present invention and generally consists of a guanosine nucleotide connected to the mRNA via an unusual 5' to 5' triphosphate linkage. The guanosine nucleotide is methylated on the 7-position directly after capping in vivo by a methyl transferase. It is referred to as a 7-methylguanylate cap, abbreviated m7G. Further modifications include the possible methylation of the 2' hydroxy-groups of the first 2 ribose sugars of the 5' end of the mRNA.

For an efficient translation of a given mRNA, such as e.g. the modified mRNA according to the invention, an effective binding of ribosomes to the ribosome binding site, which is also referred to as "Kozak sequence" (5'-GCCGCCACCAUGG, wherein the AUG denotes the start codon) is necessary. In this regard it has been established that an increased A/U content around this site permits a more efficient ribosome binding to the mRNA (Kozak, Mol Cell Biol. 1989 Nov;9(11):5073-80). Accordingly, the inventive modified mRNA may comprise a Kozak sequence for more efficient ribosome binding to the mRNA. The inventive modified mRNA may further comprise a poly-A tail, which is a sequence of up to 200 adenosine nucleotides located at the 3' end of the mRNA.

According to a further embodiment of the present invention, the inventive modified mRNA may comprise in the 3' non-translated region one or more stabilisation sequences that are capable of increasing the half-life of the mRNA in the cytosol. These stabilisation sequences may exhibit a 100% sequence homology with naturally occurring sequences that are present in viruses, bacteria and eukaryotic cells, but may however also be partly or completely synthetic. As an example of stabilising sequences that may be used in the present invention, the non-translated sequences (UTR) of the β-gtobin gene, for example of homo sapiens or xenopus laevis, may be mentioned. Another example of a stabilisation sequence has the general formula (C/U)CCANxCCC(U/A)PyxUC(C/U)CC, which is contained in the 3'UTR of the very stable mRNA that codes for α-globin, α-(I)-collagen, 15-lipoxygenase or for tyrosine hydroxylase (c.f. Holcik et al., Proc. Natl. Acad. Sci. USA 1997, 94: 2410 to 2414). Such stabilisation sequences may be used individually or in combination with one another for stabilizing the inventive modified mRNA as well as in combination with other stabilisation sequences known to the person skilled in the art.

According to a preferred embodiment, the present invention provides for a pharmaceutical composition comprising a modified mRNA according to one or more of the above embodiments of the present invention, or the inventive modified mRNA according to one or more of the above embodiments. The pharmaceutical composition optionally comprises one or more pharmaceutically acceptable excipients, carriers, diluents and/or vehicles.

The term "pharmaceutically acceptable" as used in connection with the pharmaceutical compositions of the invention, refers to molecular entities and compositions that are physiologically tolerable and do not typically produce untoward reactions when administered to an individual, such as e.g. a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" as used in connection with the pharmaceutical compositions of the invention refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 18th Edition. The term "pharmaceutically acceptable" refers to the non-toxicity of a material which does not interact with the action of the active component, i.e. with the action of the inventive modified mRNA of the pharmaceutical composition. Suitable excipients and/or vehicles for use with the pharmaceutical composition of the present invention are, for example, water, saline, dextrose, glycerol, ethanol, or the like, and combinations thereof. In addition, if desired, the vehicle may contain minor amounts of auxiliary substances such as wetting or emulsifying agents or pH buffering agents. Actual methods of preparing such dosage forms are known, or will be apparent, to those skilled in the art, such as those, e.g. which are disclosed in e.g., Remington's Pharmaceutical Sciences, Mack Publishing Company, Easton, Pennsylvania, 17th edition, 1985; Remington: The Science and Practice of Pharmacy, A.R. Gennaro, (2000) Lippincott, Williams & Wilkins. A non-limiting list of commonly used and accepted excipients is also provided in "VOLUME 3B Guidelines Medicinal products for human use Safety, environment and information Excipients in the label and package leaflet of medicinal products for human use, July 2003" issued by the European Commission, which is hereby incorporated in its entirety into the disclosure of this invention. The composition or formulation to be administered will, in any event, contain a quantity of the agent adequate to achieve the desired state in the subject being treated.

Possible carrier substances for use with the pharmaceutical composition of the present invention for parenteral administration are e.g. sterile water, sterile sodium chloride solution, polyalkylene glycols, hydrogenated naphthalenes and, in particular, biocompatible lactide polymers, lactide/glycolide copolymers or polyoxyethylene/polyoxypropylene copolymers. The pharmaceutical composition according to may further comprise filler substances or substances such as lactose or mannitol. Preferred carriers for use in the inventive pharmaceutical composition are typically aqueous carrier materials, water for injection (WFI) or water buffered with phosphate, citrate, HEPES or acetate etc. being used, and the pH is typically adjusted to 5.0 to 8.0, preferably 6.5 to 7.5. The carrier or the vehicle will additionally preferably comprise salt constituents, e.g. sodium chloride, potassium chloride or other components which render the solution e.g. isotonic. Furthermore, the carrier or the vehicle can contain, in addition to the abovementioned constituents, additional components, such as human serum albumin (HSA), polysorbate 80, sugars or amino acids.

In a more preferred embodiment the present invention provides for a pharmaceutical composition comprising the modified mRNA according to one or more embodiments of the inventive method for use in the treatment of diseases amenable to protein replacement therapy, e.g. for the treatment of hereditary or endocrinological diseases, preferably for use in the treatment of diseases caused by amino acid disorders, carbohydrate metabolism disorders, cholesterol biosynthesis disorders, fatty acid oxidation defects and fat metabolism disorders, lactic acidosis, glycogen storage diseases, mitochondrial disorders, organic acid disorders, urea cycle disorders, lysosomal storage disease disorders.

The term "protein replacement therapy" as used herein refers to the introduction of an mRNA, such as e.g. the modified of the invention into an individual having a deficiency in a protein encoded by the modified mRNA according to the invention, i.e. an mRNA modified by the inventive method of targeted modulation of the immune response against said mRNA coding for at least one biologically active polypeptide or protein.

Accordingly, the pharmaceutical composition of the invention, which comprises the modified mRNA according to one or more embodiments of the inventive method may be used in the treatment of diseases or in aiding in the treatment of the diseases which are characterized by a protein deficiency.

The term "diseases which are characterized by a protein deficiency " as used in the context of the present invention, such as e.g. in the context of the pharmaceutical composition comprising the modified mRNA of the invention refers to any disorder that presents with a pathology caused by absent or insufficient amounts of a protein. This term encompasses protein folding disorders, *i.e.,* conformational disorders, that result in a biologically inactive protein product. Protein insufficiency can be involved in infectious diseases, immunosuppression, organ failure, glandular problems, radiation illness, nutritional deficiency, poisoning, or other environmental or external insults.

There are currently about 1100 known inherited disorders characterized by protein deficiency or loss-of-function in specific tissue. These disorders may be treatable by protein replacement therapy in theory, e.g. the method of the present invention contemplates therapy for proteins currently suited for use in protein replacement therapy that is available now or will be in the future. In such disorders, certain cells or all of the cells of an individual lack a sufficient functional protein, contain an inactive form of the protein or contain insufficient levels for biological function.

Further, the list of diseases identified as being conformational disorders, caused by mutations that alter protein folding and retardation of the mutant protein in the ER, resulting in protein deficiency, is increasing. These include cystic fibrosis, α1-antitrypsin deficiency, familial hypercholesterolemia, Fabry disease, Alzheimer's disease (Selkoe, Annu. Rev. Neurosci. 1994; 17:489-517), osteogenesis imperfecta (Chessler et al., J. Biol. Chem. 1993; 268:18226-18233), carbohydrate-deficient glycoprotein syndrome (Marquardt et al., Eur. J. Cell. Biol. 1995; 66: 268-273), Maroteaux-Lamy syndrome (Bradford et al., Biochem. J. 1999; 341:193-201), hereditary blindness (Kaushal et al., Biochemistry 1994; 33:6121-8), Glanzmann thrombasthenia (Kato et al., Blood 1992; 79:3212-8), hereditary factor VII deficiency (Arbini et al., Blood 1996; 87:5085-94), oculocutaneous albinism (Halaban et al., Proc. Natl. Acad. Sci. USA. 2000; 97:5889-94) and protein C deficiency (Katsumi, et al., Blood 1996; 87:4164-75). Recently, one mutation in the X-linked disease adrenoleukodystrophy (ALD), resulted in misfolding of the defective peroxisome transporter which could be rescued by low-temperature cultivation of affected cells (Walter et al., Am J Hum Genet 2001;69:35-48). It is generally accepted that mutations take place evenly over the entire sequence of a gene. Therefore, it is predictable that the phenotype resulting from misfolding of the deficient protein exists in many other genetic disorders.

Many of the inherited protein deficient disorders are enzyme deficiencies. As indicated above, a large class of inherited-enzyme disorders involves mutations in lysosomal enzymes and are referred to as lysosomal storage disorders (LSDs). Lysosomal storage disorders are a group of diseases caused by the accumulation of glycosphingolipids, glycogen, mucopolysaccharides Examples of lysosomal disorders include but are not limited to Gaucher disease (Beutler et al., The Metabolic and Molecular Bases of Inherited Disease, 8th ed. 2001 Scriver et al., ed. pp. 3635-3668, McGraw-Hill, New York), GM1-gangliosidosis (id. at pp 3775-3810), fucosidosis (The Metabolic and Molecular Bases of Inherited Disease 1995. Scriver, C. R., Beaudet, A. L., Sly, W. S. and Valle, D., ed pp. 2529-2561, McGraw-Hill, New York), mucopolysaccharidoses (id. at pp 3421-3452), Pompe disease (id. at pp. 3389-3420), Hurler-Scheie disease (Weismann et al., Science 1970; 169, 72-74), Niemann-Pick A and B diseases, (The Metabolic and Molecular Bases of Inherited Disease 8th ed. 2001. Scriver et al. ed., pp 3589-3610, McGraw-Hill, Now York), and Fabry disease (id. at pp. 3733-3774).

Fabry disease is an X-linked inborn error of glycosphingolipid metabolism caused by deficient lysosomal α-galactosidase A (a-Gal A) activity (Desnick et al., The Metabolic and Molecular Bases of Inherited Disease, 8th Edition Scriver et al. ed., pp. 3733-3774, McGraw-Hill, New York 2001; Brady et al., N. Engl. J. Med. 1967; 276,1163-1167). This enzymatic defect leads to the progressive deposition of neutral glycosphingolipids with α-galactosyl residues, predominantly globotriaosylceramide (GL-3), in body fluids and tissue lysosomes. The frequency of the disease is estimated to be about 1 :40,000 in males, and is reported throughout the world within different ethnic groups. In classically affected males, the clinical manifestations include angiokeratoma, acroparesthesias, hypohidrosis, and characteristic corneal and lenticular opacities (The Metabolic and Molecular Bases of Inherited Disease, 8th Edition 2001, Scriver et al., ed., pp. 3733-3774, McGraw-Hill, New York). The affected male's life expectancy is reduced, and death usually occurs in the fourth or fifth decade as a result of vascular disease of the heart, brain, and/or kidneys. In contrast, patients with the milder "cardiac variant" normally have 5-15% of normal α-Gal A activity, and present with left ventricular hypertrophy or a cardiomyopathy. These cardiac variant patients remain essentially asymptomatic when their classically affected counterparts are severely compromised. Recently, cardiac variants were found in 11% of adult male patients with unexplained left ventricular hypertrophic cardiomyopathy, suggesting that Fabry disease may be more frequent than previously estimated (Nakao et aL, N. Engl. J. Med. 1995; 333: 288-293). The α-Gal A gene has been mapped to Xq22, (Bishop et al., Am. J. Hum. Genet. 1985; 37: A144), and the full-length cDNA and entire 12-kb genomic sequences encoding α-Gal A have been reported (Calhoun et al., Proc. Natl. Acad Sci. USA 1985; 82: 7364-7368; Bishop et al., Proc. Natl. Acad. Sci. USA 1986; 83: 4859-4863; Tsuji et al., Bur. J. Biochem. 1987; 165: 275-280; and Kornreich et al., Nucleic Acids Res. 1989;17: 3301-3302). There is a marked genetic heterogeneity of mutations that cause Fabry disease (The Metabolic and Molecular Bases of Inherited Disease, 8th Edition 2001, Scriver et al., ed, pp. 3733-3774, McGraw-Hill, New York.; Eng et al., Am. J. Hum. Genet. 1993; 53: 1186-1197; Eng et al., Mol. Med. 1997; 3:174-182; and Davies et al., Bur. J. Hum. Genet. 1996; 4: 219-224). To date, a variety of missense, nonsense, and splicing mutations, in addition to small deletions and insertions, and larger gene rearrangements have been reported.

Gaucher disease is a deficiency of the lysosomal enzyme β-glucocerebrosidase that breaks down fatty glucocerebrosides. The fat then accumulates, mostly in the liver, spleen and bone marrow. Gaucher disease can result in pain, fatigue, jaundice, bone damage, anemia and even death. There are three clinical phenotypes of Gaucher disease. Patients with, Type 1 manifest either early in life or in young adulthood, bruise easily and experience fatigue due to anemia, low blood platelets, enlargement of the liver and spleen, weakening of the skeleton, and in some instances have lung and kidney impairment. There are no signs of brain involvement. In Type II, early-onset, liver and spleen enlargement occurs by 3 months of age and there is extensive brain involvement. There is a high mortality rate by age 2. Type III is characterized by liver and spleen enlargement and brain seizures. The β-glucocerebrosidase gene is located on the human 1q21 chromosome. Its protein precursor contains 536 amino acids and its mature protein is 497 amino acids long.

Gaucher disease is considerably more common in the descendants of Jewish people from Eastern Europe (Ashkenazi), although individuals from any ethnic group may be affected. Among the Ashkenazi Jewish population, Gaucher disease is the most common genetic disorder, with an incidence of approximately 1 in 450 persons. In the general public, Gaucher disease affects approximately 1 in 100,000 persons. According to the National Gaucher Foundation, 2,500 Americans suffer from Gaucher disease.

Glucose-6-phosphate dehydrogenase (G6PD) deficiency is the most common X-linked human enzyme deficiency. The G6PD enzyme catalyzes an oxidation/reduction reaction that is essential for the production of ribose, which is an essential component of both DNA and RNA. G6PD also involved in maintaining adequate levels of NADPH inside the cell. NADPH is a required cofactor in many biosynthetic reactions. Individuals with this deficiency have clinical symptoms including neonatal jaundice, abdominal and/or back pain, dizziness, headache, dyspnea (irregular breathing), and palpitations.

Additional examples of diseases amenable to protein replacement therapy, which may be treated with the pharmaceutical composition of the present invention, e.g. a pharmaceutical composition comprising a modified mRNA according to one or more of the embodiments of the present invention, include
lysosomal storage disease disorders, such as, e.g. activator deficiency/GM2, gangliosidosis, alpha-mannosidosis, aspartylglucosaminuria, cholesteryl ester storage disease, chronic Hexosaminidase A deficiency, cystinosis, Danon disease, Farber disease, fucosidosis, galactosialidosis, Gaucher DiseaseType I, II, III; GM1 gangliosidosis, Infantile/late infantile/juvenile/adult/chronic I-Cell disease/Mucolipidosis II, infantile Free Sialic Acid Storage Disease/ISSD, juvenile hexosaminidase A deficiency, Krabbe disease, infantile onset/late onset lysosomal acid lipase deficiency, early onset/ late onset metachromatic Leukodystrophy, mucopolysaccharidoses disorders, pseudo-Hurler polydystrophy/Mucolipidosis IIIA, MPSI Hurler Syndrome, MPSI Scheie Syndrome, MPS I Hurler-Scheie Syndrome, MPS II Hunter syndrome, Sanfilippo syndrome Type A/MPS III A, Sanfilippo syndrome Type B/MPS III B, Sanfilippo syndrome Type C/MPS III C, Sanfilippo syndrome Type D/MPS III D, Morquio Type A/MPS IVA, Morquio Type B/MPS IVB, MPS IX Hyaluronidase Deficiency, MPS VI Maroteaux-Lamy, MPS VII Sly Syndrome, Mucolipidosis I/Sialidosis, Mucolipidosis IIIC, Mucolipidosis type IV, Multiple sulfatase deficiency, Niemann-Pick Disease, Type A/Type B/Type C neuronal ceroid lipofuscinoses, CLN6 disease, atypical late infantile/late onset variant/early juvenile Batten-Spielmeyer-Vogt/Juvenile NCL/CLN3 disease, Finnish Variant Late Infantile CLN5, Jansky-Bielschowsky disease/Late infantile CLN2/TPP1 Disease, Kufs/Adult-onset NCL/CLN4 disease, Northern Epilepsy/variant late infantile CLN8, Santavuori-Haltia/Infantile CLN1/PPT disease, beta-mannosidosis, Pompe disease/Glycogen storage disease type II, pycnodysostosis, Sandhoff disease/Adult Onset/GM2 gangliosidosis, Sandhoff disease/GM2 gangliosidosis - Infantile, Sandhoff disease/GM2 gangliosidosis - Juvenile, Schindler disease, Salla disease/Sialic Acid Storage Disease, Tay-Sachs/GM2 gangliosidosis, Wolman disease, and/or
amino acid metabolism disorders, such as, e.g. alkaptonuria, aspartylglucosaminuria, methylmalonic acidemia, maple syrup urine disease, homocystinuria, tyrosinemia, trimethylaminuria, Hartnup disease, biotinidase deficiency, ornithine carbamoyltransferase deficiency, carbamoyl-phosphate synthase I deficiency disease, citrullinemia, hyperargininemia, hyperhomocysteinemia, hypermethioninemia, hyperlysinemias, nonketotic hyperglycinemia, propionic acidemia, hyperprolinemia, and/or
carbohydrate metabolism disorders, such as e.g. lactose intolerance, other disorders of carbohydrate metabolism, glycogen storage disease, glycogen storage disease type I (von Gierke's disease), glycogen storage disease type II (Pompe's disease), glycogen storage disease type III, glycogen storage disease type IV, glycogen storage disease type V (McArdle's disease), disorders of fructose metabolism, essential fructosuria, fructose-1,6-diphosphatase deficiency, hereditary fructose intolerance, disorders of galactose metabolism,
galactosaemia, galactokinase deficiency, disorders of intestinal carbohydrate absorption, e.g. glucose-galactose malabsorption, sucrase deficiency, disorders of pyruvate metabolism and gluconeogenesis, e.g. deficiency of phosphoenolpyruvate carboxykinase, deficiency of pyruvate carboxylase, deficiency of pyruvate dehydrogenase, other disorders of carbohydrate metabolism, such as, e.g. essential pentosuria, oxalosis, oxaluria, renal glycosuria, and/or
lipid and cholesterol biosynthesis disorders, such as, e.g. pure hypercholesterolemia, familial hypercholesterolemia, Fredrickson's hyperlipoproteinaemia, type IIa, hyperbetalipoproteinaemia, hyperlipidaemia, group A, low-density-lipoprotein-type (LDL) hyperlipoproteinaemia, hyperglyceridaemia, endogenous hyperglyceridaemia, Fredrickson's hyperlipoproteinaemia, type IV, hyperlipidaemia, group B, hyperprebetalipoproteinaemia
very-low-density-lipoprotein-type (VLDL) hyperlipoproteinaemia, and/or
lactic acidosis caused by e.g. glucose-6-phosphatase deficiency, fructose 1,6-diphosphatase deficiency, pyruvate dehydrogenase deficiency, pyruvate carboxylase deficiency, and/or
glycogen storage diseases (GSDs), e.g. GSD type I, GSD type II, GSD type III, GSD type IV, GSD type V, GSD type VI, GSD type VII, GSD type VIII, GSD type IX, GSD type X, GSD type XI, and/or
mitochondrial disorders, such as, e.g. Kearns-Sayre syndrome, mitochondrial encephalopathy, lactic acidosis and stroke-like episodes (MELAS syndrome), mitochondrial neurogastrointestinal encephalopathy syndrome (MNGIE), myoclonus with epilepsy and with ragged red fibers (MERRF syndrome), neuropathy, ataxia, and retinitis pigmentosa (NARP syndrome), and/or
organic acid disorders, such as, e.g. glutaric acidemia type 1, type 2, hyperlysinemia, pipecolic acidemia, saccharopinuria, and/or urea cycle disorders, such as, e.g. citrullinemia, hyperammonemia.

In addition to inherited disorders, the pharmaceutical composition according to the invention may be used in and/or used in aiding in the treatment of other enzyme deficiencies, which arise from damage to a tissue or organ resulting from primary or secondary disorders. For example, damaged pancreatic tissue, or pancreatitis, is caused by alcoholism results in a deficiency in pancreatic enzymes necessary for digestion. Pancreatitis is currently being treated using enzyme replacement therapy.

In addition to disorders characterized by protein deficiencies, some disorders may be treated by the pharmaceutical composition of the present invention to replace proteins in order to enhance or stimulate biological processes. For example, currently individuals with anemia are administered recombinant erythropoietin (EPOGEN®, PROCRIT®, EPOIETIN®) to stimulate red blood cell production and increase oxygen transportation to tissues. In addition, recombinant interferons such as interferon alpha 2b (INTRON A®, PEG-INTRON®, or REBETOL®), and interferon beta 1a (AVONEX®, BETASERON®) are administered to treat hepatitis B and multiple sclerosis, respectively. Still other proteins administered are recombinant human deoxyribonuclease I (rhDNase- PULMOZYME®), an enzyme which selectively cleaves DNA used to improve pulmonary function in patients with cystic fibrosis; recombinant thyroid stimulating hormone (THYROGEN®) developed for use in thyroid cancer patients who have had near-total or total thyroidectomy, and who must therefore take thyroid hormones; recombinant G-CSF (NEUPOGEN®) for treating neutropenia from chemotherapy, and digestive enzymes in individuals with pancreatitis. Thus, the pharmaceutical composition of the present invention may be used in the treatment of the above mentioned conditions.

Additionally, the the pharmaceutical composition according to the invention may be used in the treatment of and/or used in aiding in the treatment of growth hormone deficiency, such as e.g. pituitary-related growth hormone deficiency, growth hormone releasing hormone (GHRH) deficiency. Accordingly, the pharmaceutical composition according to the present invention may be used in treating or aiding in the treatment of the above diseases.

The pharmaceutical composition of the invention comprising the modified mRNA according to one or more embodiments of the inventive method may also be used in another area of protein replacement therapy, such as, e.g. in the treatment of infectious diseases and cancer with antibodies, which have a highly specific, well-defined active site. Accordingly, the pharmaceutical composition of the invention may comprise a modified mRNA of the invention, which codes for an antibody for use in the treatment of cancer or infectious diseases. The antibodies encoded by the modified mRNA of the invention comprised in the pharmaceutical composition may be e.g. any type of antibody, however, in a preferred embodiment the encoded antibody is a single chain Fv fragments (scFv), preferably an intrabody.

The term "intrabody" or "intrabodies" as used herein refers to intracellularly expressed antibodies. For example, whole antibodies, heavy chains, Fab' fragments, single chain antibodies and diabodies can be used as intrabodies, preferably the intrabody is a single chain antibody.

The pharmaceutical composition comprising a modified mRNA of the invention, which codes for an antibody, such as, e.g. a single-chain Fv fragments (scFv) may be administered to a patient in need thereof, preferably a therapeutically effective amount of the pharmaceutical composition is administered. The cancer can include, e.g. breast, colon, ovarian, endometrial, gastric, pancreatic, prostate and salivary gland cancer. The administration of the pharmaceutical composition of the invention can be by any of a variety of convenient methods including, e.g. systemic injectable administration, injection into a tumor or cancerous tissue, oral administration.

According to another embodiment the present invention provides a method of treating a subject in need of protein replacement therapy comprising administering to a subject in need thereof a pharmaceutically effective amount of the pharmaceutical composition according to one or more embodiments of the present invention or an effective amount of the modified mRNA, which has been modified in accordance to one or more of the embodiments of the inventive method.

The mode and method of administration and the dosage of the pharmaceutical composition according to the invention depends on several factors, such as e.g. on the nature of the disease to be cured, also the body weight, the age and the sex of the patient and the route of administration of the inventive pharmaceutical composition.

The pharmaceutical composition according to the present invention composition may be administered to an individual in need thereof, for example, a patient, by any suitable administration route, such as by oral, topical, rectal, vaginal, dermal, intra-tumoural, nasal, lingual, parenteral administration or administration by inhalation, insufflation, injection, infusion or by enema. Thus, the modified mRNA obtainable by one or more embodiments of the inventive method for targeted modulation of the immunogenicity and/or immunostimulatory capacity of said mRNA, the pharmaceutical composition according to the present invention may be adapted, for example, for oral, topical, rectal, vaginal, dermal, intra-tumoural, nasal, lingual, parenteral administration or administration by inhalation, insufflation, injection, infusion or by enema.

Furthermore, the method of treatment according to the present invention may comprise oral, topical, rectal, vaginal, dermal, intra-tumoural, nasal, lingual, parenteral administration or administration by inhalation, insufflation, injection, infusion or by enema. Preferred administration routes are oral and parenteral administration, such as intravenous, intramuscular, subcutaneous, intranodal, intralymphatic, intra-tumoural or intraperitoneal injection or transdermal delivery.

More preferably, the present invention provides a method for expressing a biologically active peptide, polypeptide or protein in a tissue *in vivo,* the method comprising contacting the patient with a pharmaceutical composition according to one or more of the above embodiments of the invention, or contacting the patient with the inventive modified mRNA according to any one of the above embodiments of the invention, wherein administering the pharmaceutical composition or the modified mRNA results in a reduced innate immune response by the patient relative to a patient contacted with the wild type mRNA molecule encoding the same polypeptide or protein. Typically, the level of mRNA expression in vivo is increased by the modified target mRNA of the invention as compared to the wild type mRNA.

Further embodiments of the present invention are provided by the items as specified below:
item 22. Modified mRNA that codes for at least one biologically active peptide polypeptide or protein, wherein
   - the cytosine-content of the coding region of the modified mRNA is larger than the cytosine-content of the coding region of the wild type mRNA coding for the polypeptide or protein whereby the encoded amino acid sequence is unchanged compared to the wild type sequence and optionally
   - if no cytosine is present in any of the at least one codon coding for the amino acid, the at least one codon of the wild type sequence that codes for a relatively rare tRNA in the cell is exchanged for a codon that codes for a relatively frequent tRNA in the cell that carries the same amino acid as the relatively rare tRNA.
item 23. Modified mRNA according to item 22, wherein all codons of the wild type sequence, which code for a relatively rare codon in the cell, have been replaced by codons which code a relatively frequent tRNA in the cell, which carries the same amino acid as the relatively rare tRNA.
item 24. Modified mRNA according to item 22 or item 23, wherein the cytosine-content of the polypeptide- or protein-coding region of the modified mRNA is at least 10%, preferably at least 12.5%, more preferred at least 15% greater than the cytosine content compared to the polypeptide or protein coding region of the wild type mRNA.
item 25. Modified mRNA according to any one of items 22 - 24, wherein the coding region of the modified mRNA is modified such that a maximal cytosine-content is achieved by means of codons which encode relatively frequent tRNAs.
item 26. Modified mRNA according to any one of items 22 - 25, wherein the codon adaptation index (CAI) of the region coding for the polypeptide or protein is greater by at least 0.05, preferably greater by at least 0.1, preferably greater by at least 0.125, more preferred greater by at least 0.15 than the CAI of the wild type region of the mRNA coding for the polypeptide or protein.
item 27. Modified mRNA according to any one of items 22 - 26, wherein the coding region of the modified mRNA and/or the 5' and/or 3' untranslated region of the modified mRNA is changed in comparison to the polypeptide- or protein-encoding wildtype region such that no destabilizing elements are present.
item 28. Modified mRNA according to any one of items 22 - 27, wherein the modified mRNA is characterized by having a 5' CAP structure and/or a polyA-tail of at least 70 nucleotides and/or a 3' stabilizing sequence.
item 29. Modified mRNA according to any one of items 22 - 28, wherein the modified mRNA coding for the polypeptide or protein is characterized by a lower binding affinity to mammalian cellular RNA sensors than the wild type sequence coding for the polypeptide or protein, wherein the mammalian cellular RNA sensors comprise one or more of TLR3, TLR7, TLR8, PKR, MDA5, RIG-I, LGP2, 2'-5'-oligoadenylate synthetase
item 30- Modified mRNA according to any one of items 22 - 29, wherein the modified mRNA encodes a polypeptide or protein for use in protein replacement therapy.
item 31. Modified mRNA according to any one of items 22 - 30, wherein the modified mRNA encodes for a polypeptide or protein selected from the group of α - glucosidase,
   acid β-glucosidase , α-galactosidase A, galactocerebrosidase Acid α-Mannosidase, Acid β-Mannosidase, Acid α-L-fucosidase, α-N-Acetylgalactosaminidase, β-Hexosaminidase A, β-Hexosaminidase B, α-L-Iduronidase, β-Glucuronidase, Sialidase, Iduronate sulfatase, Acid sphingomyelinase, pituitary-related growth hormone, growth hormone releasing hormone (GHRH), erythropoetin, or thyroid stimulating hormone.
item 32. Modified mRNA according to any one of items 22 - 31, for use in protein replacement therapy.
item 33. Kit of parts for use in aiding in protein replacement therapy, wherein the kit comprises
   (i) the isolated and purified mRNA according to any one of items 22 - 31,
   (ii) means for applying the mRNA to an individual, e.g. a human.

**SEQUENCE LISTING**

| | |
|---|---|
| SEQ ID NO:1 | R873, *Photinus pyralis* luciferase wild type mRNA |
| SEQ ID NO:2 | R875, G/C-enriched mRNA sequence |
| SEQ ID NO:3 | R2103, C-enriched mRNA sequence |
| SEQ ID NO:4 | R2349, G/C-enriched mRNA sequence |
| SEQ ID NO:5 | R2350, C-optimized mRNA sequence |
| SEQ ID NO:6 | R2791, G/C-enriched mRNA sequence |
| SEQ ID NO:7 | R2793, C-optimized mRNA sequence |

### EXAMPLES

### Example 1: Preparation of mRNA

### Preparation of DNA and mRNA constructs

For the present examples DNA sequences encoding the *Photinus pyralis* luciferase were prepared and used for subsequent *in vitro* transcription reactions.

According to a first preparation, the DNA sequences coding for the mRNAs shown in Table 1 were prepared.

The G/C-enriched sequences of the examples provided were obtained according to the method as disclosed in WO2002098443 A2. The C-enriched modified mRNA coding region was obtained by the method of the present invention. However, alternatively, the C-enriched mRNA of the examples included may also be obtained according to the alternative embodiment as disclosed above, i.e. in a first step, the G/C content may be increased, e.g. by substituting wild type codons exhibiting a lower content of G and C nucleotides as disclosed in WO2002098443 A2. As a second step the G/C-enrichment or maximization is followed by a step of further C-optimization.

A vector for *in vitro* transcription was constructed containing a T7 promoter followed by a sequence coding for *Photinus pyralis* luciferase (PpLuc(wt), obtained from Promega) and a poly(A) sequence of 70 adenosine nucleotides (A70 poly(A) sequence). mRNA obtained from this vector by *in vitro* transcription is designated as "PpLuc(wt) - A70" (R873). In Figure 1 (SEQ ID NO: 1) the sequence of the corresponding wild type luciferase mRNA is shown.

The vector was modified by replacing the wild type coding sequence of the mRNA by a GC-enriched (R875, Figure 2, SEQ ID NO:2) or C-enriched (R2103, Figure 3, SEQ ID NO:3) coding sequence, respectively, for stabilization. mRNA was obtained from these vectors by in vitro transcription.

A further vector for *in vitro* transcription was constructed containing a T7 promoter followed by a GC-enriched sequence coding for *Photinus pyra*/*is* luciferase (PpLuc(GC)III) and an A64 poly(A) sequence. The vector was modified by replacing the GC-optimized coding sequence by a C-enriched sequence.

mRNA obtained from these vectors by in vitro transcription is designated as "PpLuc(GC)III - A64" (R2349) or PpLuc(GC)V - A64 (R2350), respectively.
In SEQ ID NO: 4 (Figure 4) and SEQ ID NO: 5 (Figure 5) the sequences of the corresponding mRNAs are shown.

Two further vectors were prepared by introducing a 5'-TOP-UTR derived from the ribosomal protein 32L, modifying the wild type coding sequence by introducing a GC-optimized or C-optimized sequence for stabilization, followed by a stabilizing sequence derived from the albumin-3'-UTR, a stretch of 64 adenosines (poly(A)-sequence), a stretch of 30 cytosines (poly(C)-sequence), and a histone stem loop. mRNA obtained from these vectors by *in vitro* transcription is designated as R2791 or R2793, respectively. In SEQ ID NO: 6 (Figure 6) and SEQ ID NO: 7 (Figure 7) the sequences of the corresponding mRNAs are shown.

**Table 1: Luciferase mRNA constructs**

| R number | SEQ ID NO. | Construct | Composition of complete RNA | | |
|---|---|---|---|---|---|
| R873 | SEQ ID NO. 1 | ppLuc(wt)..A70 | Base | Number | % |
| | | | | | |
| | | | A | 534 | 30.4 |
| | | | C | 371 | 21.1 |
| | | | G | 418 | 23.8 |
| | | | T | 435 | 24.7 |
| R875 | SEQ ID NO. 2 | ppLuc(GC)II..A70 | Base | Number | % |
| | | | | | |
| | | | A | 405 | 23.0 |
| | | | C | 579 | 32.9 |
| | | | G | 517 | 29.4 |
| | | | T | 257 | 14.6 |
| R2103 | SEQ ID NO. 3 | ppLuc(GC)V..A70 | Base | Number | % |
| | | | | | |
| | | | A | 390 | 22.2 |
| | | | C | 710 | 40.4 |
| | | | G | 386 | 22.0 |
| | | | T | 272 | 15.5 |
| R2349 | SEQ ID NO. 4 | PpLuc(GC)III..A64 | Base | Number | % |
| | | | | | |
| | | | A | 397 | 22.7 |
| | | | C | 600 | 34.4 |
| | | | G | 492 | 28.2 |
| | | | T | 257 | 14.7 |
| R2350 | SEQ ID NO. 5 | PpLuc(GC)V..A64 | Base | Number | % |
| | | | | | |
| | | | A | 382 | 21.9 |
| | | | C | 709 | 40.6 |
| | | | G | 385 | 22.1 |
| | | | T | 270 | 15.5 |
| R2791 | SEQ ID NO. 6 | 32L4..PpLuc(GC)II..albumin7..A64 ..C30-histoneSL-N5 | Base | Number | % |
| | | | | | |
| | | | A | 476 | 23.4 |
| | | | C | 668 | 32.8 |
| | | | G | 556 | 27.3 |
| | | | T | 335 | 16.5 |
| R2793 | SEQ ID NO. 7 | 32L4..PpLuc(GC)V..albumin7..A6 4..C30-histoneSL-N5 | Base | Number | % |
| | | | | | |
| | | | A | 461 | 22.7 |
| | | | C | 799 | 39.3 |
| | | | G | 425 | 20.9 |
| | | | T | 350 | 17.2 |

### 2. In vitro transcription

The respective DNA plasmids prepared according to paragraph 1 were transcribed *in vitro* using T7 polymerase in the presence of a CAP analog (m⁷GpppG). Subsequently the mRNA was purified using PureMessenger® (CureVac, Tübingen, Germany; WO2008/077592A1).

### 3. Reagents

Complexation Reagent: protamine

### 4. Formulation of mRNA

The mRNA was complexed with protamine by addition of protamine to the mRNA in the ratio RNA / protamine of 2:1 (w/w).

### Example 2: Immunostimulation of peripheral blood mononuclear cells (PBMCs) treated with modified mRNAs

### Preparation of human PBMCs

25 ml of a buffy coat were layered over 20 ml of Ficoll in a 50 ml Falcon tube. After centrifugation at 805 relative centrifugal force (rcf) for 20 minutes, cells at the interphase were collected. Cells were washed two times by resuspending in PBS and centrifuging. After counting, cells were resuspended at 50 million cells per ml in fetal calf serum, 10% DMSO, and frozen.

### PBMC stimulation

Human PBMCs were seeded at a density of 10⁶ cells/ml into each well of a 96-well plate (2 x 10⁵ cells/well) in X-Vivo 15 medium (Lonza) and treated with 10 µg/ml of GC- or C-enriched mRNA for 20 hours and the TNFa concentration was determined in the supernatant by ELISA.

### Cytokine ELISA (TNFα)

96 well ELISA plates were coated with capture antibody (BD Pharmingen) in coating buffer (15 mM Na₂CO₃, 15 mM NaHCO₃, 0.02% NaN3, pH 9.6) over night at room temperature. Plates were blocked with blocking buffer (PBS, 0.05% Tween-20, 1% BSA, pH7.4) for 1 hour at room temperature. Plates were washed three times (PBS, 0.05% Tween-20, pH7.4). 50 µl of supernatants of stimulated PBMC, diluted with 50 µl of blocking buffer, were added to the plates and incubated for 2 hours at room temperature. Plates were washed three times. Biotinylated detection antibody diluted in blocking buffer was added to the plates and incubated for 1 hour at room temperature. Plates were washed three times. HRP-Streptavidin diluted in blocking buffer without NaN3 was added to the plates and incubated for 30 minutes at room temperature. Plates were washed three times. 100 µl/well of TMB substrate (Perbioscience) was added to the plates. To stop color development, 100 µl of 20% H₂SO₄ was added to the plates. Absorbance at 450 nm was measured.

### Transfection of HeLa cells and determination of luciferase activity

HeLa cells were trypsinized and washed in opti-MEM medium (Life Technologies). Cells were electroporated with PpLuc-encoding mRNA in 200 µl volume. Electroporated cells were seeded in 24-well plates in 1 ml of RPMI 1640 medium (between 0.3 to 0.5 µg of PpLuc mRNA and 100000 cells per well). 6, 24, or 48 hours after transfection, medium was aspirated and cells were lysed. Lysates were stored at -80°C. Luciferase activity was measured as relative light units (RLU) in a BioTek SynergyHT plate reader. PpLuc activity was measured at 5 seconds measuring time using 50 µl of lysate and 200 µl of luciferin buffer (75 µM luciferin, 25 mM Glycylglycin, pH 7.8 (NaOH), 15 mM MgSO₄, 2 mM ATP).

### Results

Figure 8 shows that the treatment of human PBMCs with C-enriched mRNA (R2793) results in significantly less TNFa secretion compared to treatment with GC-enriched mRNA (R2791).

Figure 9A shows that the activity of luciferase encoded by GC-enriched mRNA (R875) and C-enriched mRNA (R2103) was comparable both in terms of peak level and kinetics.

Figure 9B shows that the activity of luciferase encoded by GC-enriched mRNA (R875) was much higher than that of the wildtype construct (R873).

### Example 3: Dose-response relationship for immunostimulation of PBMCs treated with modified mRNAs

### PBMC stimulation

Human PBMCs were prepared as described in Example 2.PBMCs were seeded at a density of 10⁶ cells/ml into each well of a 96-well plate (2 x 10⁵ cells/well) in X-Vivo 15 medium (Lonza) and treated with 40 or 20 µg/ml of GC- or C-enriched mRNAs as indicated for 20 hours. The TNFα and IFNα concentrations were determined in the supernatant by ELISA.

### Cytokine ELISA

The TNFα ELISA was performed as described in Example 2. The IFNα ELISA was performed analogously, replacing the capture and detection antibodies appropriately (Mabtech).

### Results

Figure 10 shows the dose-response relationship for TNFα secretion of human PBMCs treated with various modified mRNAs. As can be seen the treatment with C-enriched mRNA results in less TNFa secretion than with GC-enriched mRNA.

Figure 11 shows the dose-response relationship for IFN secretion of human PBMCs treated with various modified mRNAs. As can be seen the treatment with C-enriched mRNA results in less TNF secretion than with GC-enriched mRNA.

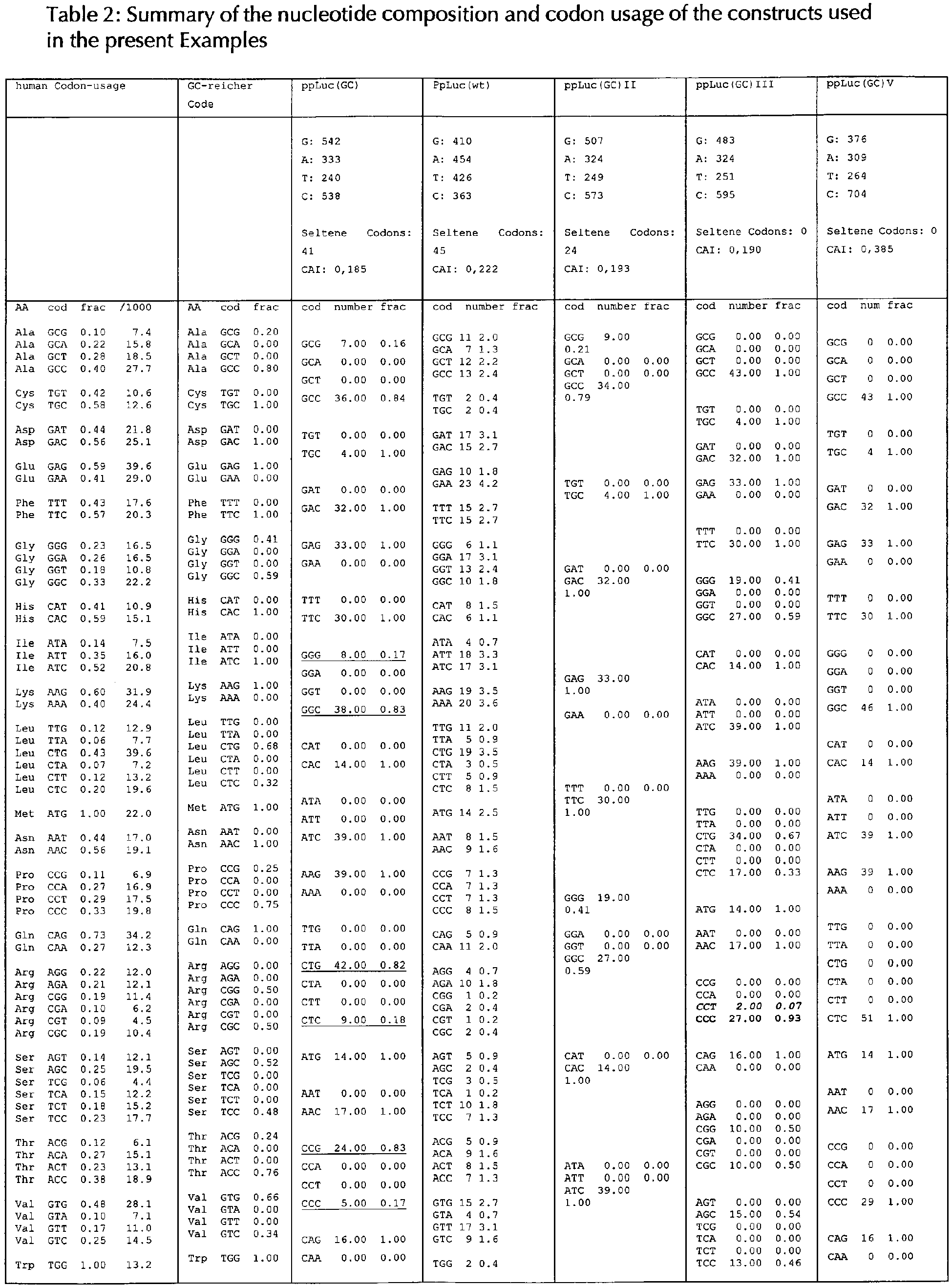

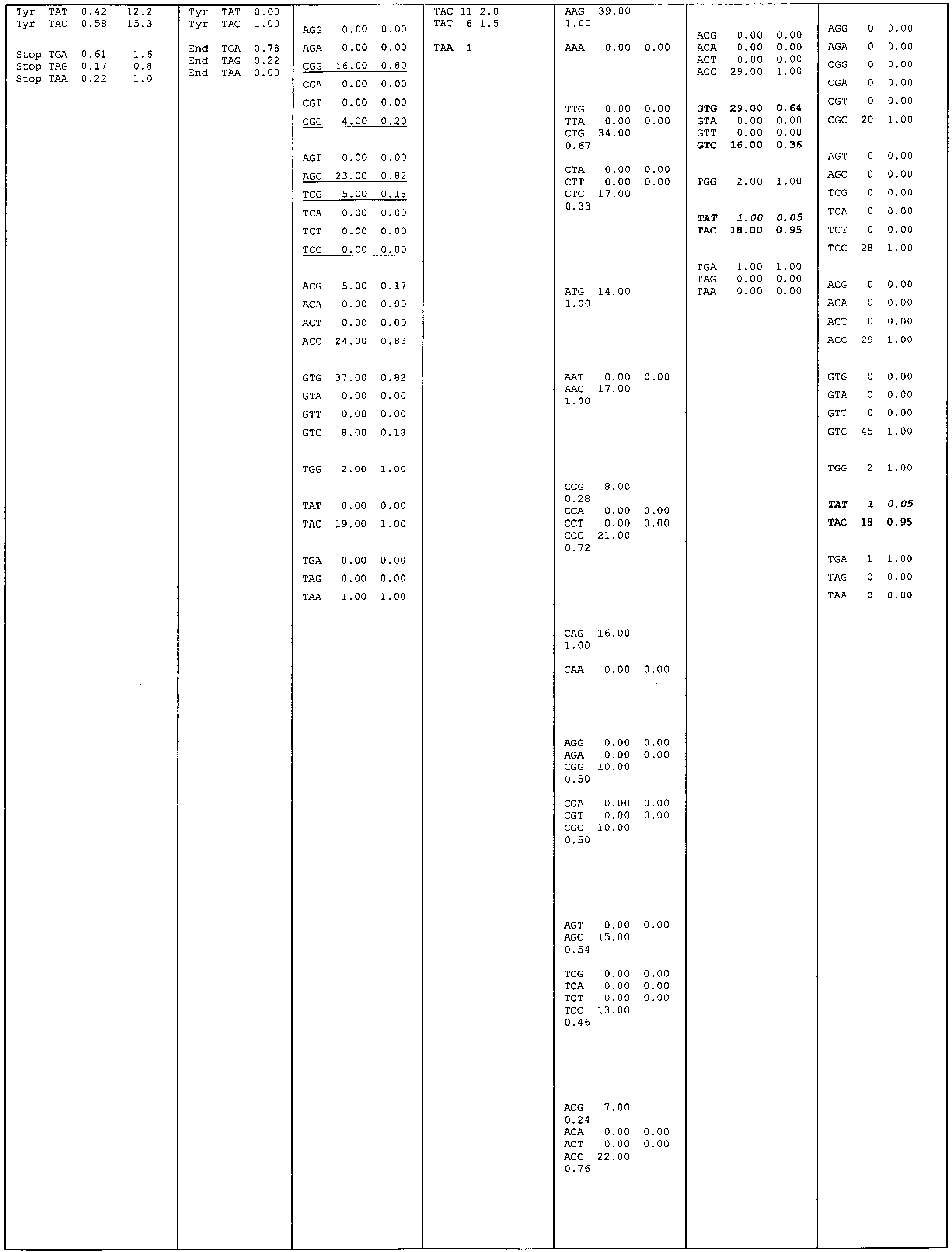

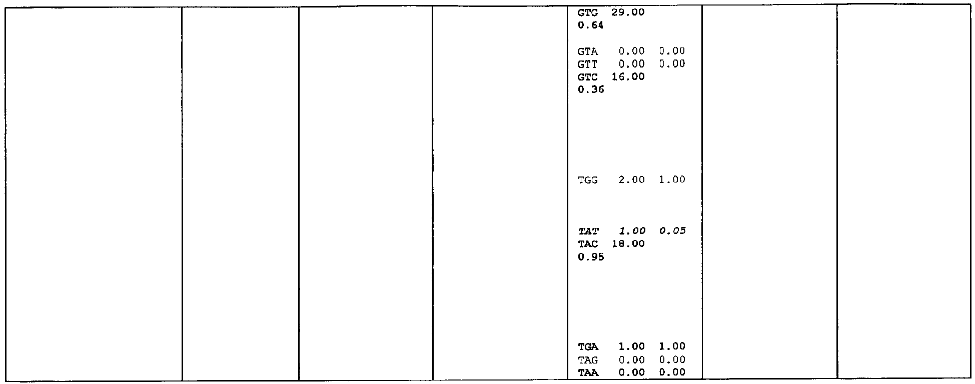

### ITEMS

1. Method for targeted modulation, preferably reduction, of the immune response against an mRNA coding for at least one biologically active polypeptide or protein, wherein the method comprises the steps of
   (1) identifying a target mRNA wild type sequence coding for the biologically active polypeptide or protein and
   (2) modifying at least 70% of the codons of the wild type sequence which are cytosine content optimizable by increasing the cytosine-content of the mRNA such that the cytosine-content of the region of the coding region of the modified mRNA is larger than the cytosine-content of the coding region of the wild type mRNA, whereby the encoded amino acid sequence is unchanged compared to the wild type sequence and, optionally
   (3) modifying at least 70% of the codons for amino acids which are not eligible for C-optimization, but are guanosine-content optimizable by increasing the guanosine-content
2. Method according to item 1, wherein the coding region of the target mRNA is modified such that a maximal cytosine-content is achieved by introduction of codons, which encode relatively frequent tRNAs.
3. Method according to item 1 or item 2, wherein the cytosine-content of the region of the modified mRNA coding for at least one the polypeptide or protein is at least 10%, preferably at least 12.5%, more preferred at least 15% greater than the cytosine-content compared to the polypeptide or protein coding region of the wild type mRNA.
4. Method according to any one of items 1-3, wherein all codons of the wild type sequence, which are not cytosine-content optimizable and which code for a relatively rare codon in the cell have been replaced by codons which code for a relatively frequent tRNA in the cell, which carries the same amino acid as the relatively rare tRNA.
5. Method according to any one of items 1 - 4, wherein the codon adaptation index (CAI) of the coding region of the modified target mRNA is greater by at least 0.05, preferably greater by at least 0.1, preferably greater by at least 0.125, more preferred greater by at least 0.15 than the CAI of the wild type region of the mRNA coding for the polypeptide or protein.
6. Method according to any one of items 1 - 5, wherein the method further comprises the step of determining the immunogenicity and/or immunostimulatory capacity of the modified target mRNA coding for the at least one polypeptide or protein.
7. Method according to any one of items 1 - 6, wherein determining the immunogenicity and/or immunostimulatory capacity of the modified target mRNA comprises the sub-steps of
   (i) transfecting PBMC with the modified mRNA according to any one of item 1 - 5,
   (ii) cultivating the cells for an appropriate amount of time, preferably at least 8 hours, preferably for at least 12h, more preferably for at least 20 hours,
   (iii) measuring the amount of pro-inflammatory cytokines in the cell supernatant.
8. Method according to any one of items 1 - 7, wherein the target mRNA of lower immunogenicity and/or immunostimulatory capacity coding for the polypeptide or protein is selected, optionally the method according to any one of items 1 - 7 is reiterated to further decrease the immunogenicity and/or immunostimulatory capacity of the target mRNA.
9. Method according to any one of items 1 - 8, wherein the method of targeted modulation of the immune response against a target mRNA encoding at least one biologically active polypeptide or protein is carried out by way of executing at least one algorithm on a computer with the aid of suitable software.
10. Method according to any one of items 1 -9, wherein the modified target mRNA is obtained by synthesis.
11. Method according to any one of items 1- 10, wherein the modified target mRNA is obtained by in vitro transcription, preferably by bacteriophage polymerase-mediated in vitro transcription, preferably by Sp6 polymerase in vitro transcription and/or T3 polymerase-mediated in vitro transcription, more preferably by T7 polymerase-mediated in vitro transcription.
12. Modified target mRNA encoding at least one biologically active polypeptide or protein obtainable by any one of items 1 - 11 having reduced immunogenicity and/or immunostimulatory capacity compared to the wild type target mRNA.
13. Modified target mRNA according to item 12, wherein the modified target mRNA is characterized by a lower affinity to one or more of TLR3, TLR7, TLR8, PKR, MDA5, RIG-I, LGP2 or 2'-5'-oligoadenylate synthetase compared to the wild type target mRNA encoding the at least one biologically active polypeptide or protein.
14. Modified target mRNA according to one of items 12 or 13, wherein the modified target mRNA is devoid of destabilising sequence elements (DSE) in the 3' and/or 5' UTR.
15. Modified target mRNA according to any one of items 12 - 14, wherein the modified target mRNA further comprises a 5' CAP structure and/or a polyA-tail of at least 60 nucleotides, preferably of at least 70 nucleotides and/or a 3' stabilizing sequence.
16. Pharmaceutical composition comprising the modified target mRNA obtainable by any one of items 1 to 11 and/or the modified target mRNA according to any one of items 12- 15, optionally comprising one or more pharmaceutically acceptable excipients, carriers, diluents and/or vehicles.
17. Pharmaceutical composition according to item 16 for use in the treatment of protein deficiency disorders by protein replacement therapy.
18. Pharmaceutical composition according to item 16 or the modified target mRNA according to any one of items 12 - 15 for use in the treatment of diseases amenable to protein replacement therapy, preferably for use in the treatment of e.g. hereditary or endocrinological dieases, selected from the group comprising amino acid metabolism disorders, carbohydrate metabolism disorders, cholesterol biosynthesis disorders, fatty acid oxidation defects and fat metabolism disorders, lactic acidosis, glycogen storage diseases, mitochondrial disorders, organic acid disorders, urea cycle disorders, vitamin/cofactor disorders, lysosomal storage disease disorders.
19. Method of treating a subject in need of protein replacement therapy comprising administering to a subject in need thereof a pharmaceutically effective amount of the pharmaceutical composition according to any one of items 16 to 18 or an effective amount of the modified target mRNA according to any one of items 12 - 15.
20. Method according to item 19, wherein the disease to be treated by protein replacement therapy is selected from the group consisting of hereditary or endocrinological diseases, wherein the group comprises amino acid metabolism disorders, carbohydrate metabolism disorders, cholesterol biosynthesis disorders, fatty acid oxidation defects and fat metabolism disorders, lactic acidosis, glycogen storage diseases, mitochondrial disorders, organic acid disorders, urea cycle disorders, vitamin/cofactor disorders, lysosomal storage disease disorders.
21. Method for expressing a biologically active polypeptide or protein in a tissue *in vivo,* the method comprising contacting the patient with the pharmaceutical composition according to any of items 16- 18 or contacting the tissue with the modified target mRNA according to any one of items 12-15, wherein administering the pharmaceutical composition or the modified target mRNA results in a reduced innate immune response by the patient relative to a patient contacted with the wild type target mRNA molecule encoding the same polypeptide or protein, wherein the level of mRNA expression *in vivo* is preferably increased by the modified target mRNA of the invention as compared to the wild type target mRNA.

SEQUENCE LISTING

<110>  CureVac AG

<120>  Modified RNA with decreased immunostimulatory properties

<130>  CU01P154WO1EPT1

<160>  12

<170>  PatentIn version 3.5

<210>  1
<211>  1758
<212>  RNA
<213>  Artificial Sequence

<220>
<223>  Photinus pyralis luciferase

<400>  1
gggagaugua caaagcuuac cauggaagac gccaaaaaca uaaagaaagg cccggcgcca      60

uucuauccgc uggaagaugg aaccgcugga gagcaacugc auaaggcuau gaagagauac     120

gcccugguuc cuggaacaau ugcuuuuaca gaugcacaua ucgaggugga caucacuuac     180

gcugaguacu ucgaaauguc cguucgguug gcagaagcua ugaaacgaua ugggcugaau     240

acaaaucaca gaaucgucgu augcagugaa aacucucuuc aauucuuuau gccgguguug     300

ggcgcguuau uuaucggagu ugcaguugcg cccgcgaacg acauuuauaa ugaacgugaa     360

uugcucaaca guaugggcau uucgcagccu accguggugu ucguuuccaa aaagggguug     420

caaaaaauuu ugaacgugca aaaaaagcuc ccaaucaucc aaaaaauuau uaucauggau     480

ucuaaaacgg auuaccaggg auuucagucg auguacacgu ucgucacauc ucaucuaccu     540

cccgguuuua augaauacga uuuugugcca gaguccuucg auagggacaa gacaauugca     600

cugaucauga acuccucugg aucuacuggu cugccuaaag gugucgcucu gccucauaga     660

acugccugcg ugagauucuc gcaugccaga gauccuauuu uuggcaauca aaucauuccg     720

gauacugcga uuuuaagugu uguuccauuc caucacgguu uuggaauguu uacuacacuc     780

ggauauuuga uauguggauu ucgagucguc uuaauguaua gauuugaaga agagcuguuu     840

cugaggagcc uucaggauua caagauucaa agugcgcugc uggugccaac ccuauucucc     900

uucuucgcca aaagcacucu gauugacaaa uacgauuuau cuaauuuaca cgaaauugcu     960

ucugguggcg cuccccucuc uaaggaaguc ggggaagcgg uugccaagag guuccaucug    1020

ccagguauca ggcaaggaua ugggcucacu gagacuacau cagcuauucu gauuacaccc    1080

gagggggaug auaaaccggg cgcggucggu aaaguuguuc cauuuuuuga agcgaagguu    1140

guggaucugg auaccgggaa aacgcugggc guuaaucaaa gaggcgaacu gugugugaga    1200

gguccuauga uuauguccgg uuauguaaac aauccggaag cgaccaacgc cuugauugac    1260

aaggauggau ggcuacauuc uggagacaua gcuuacuggg acgaagacga acacuucuuc 1320

aucguugacc gccugaaguc ucugauuaag uacaaaggcu aucagguggc ucccgcugaa 1380

uuggaaucca ucuugcucca acaccccaac aucuucgacg caggugucgc aggucuuccc 1440

gacgaugacg ccggugaacu ucccgccgcc guuguuguuu uggagcacgg aaagacgaug 1500

acggaaaaag agaucgugga uuacgucgcc agucaaguaa caaccgcgaa aaaguugcgc 1560

ggaggaguug uguuugugga cgaaguaccg aaaggucuua ccggaaaacu cgacgcaaga 1620

aaaaucagag agauccucau aaaggccaag aagggcggaa agaucgccgu guaaccucua 1680

guagaucuaa aaaaaaaaaa aaaaaaaaaa aaaaaaaaaa aaaaaaaaaa aaaaaaaaaa 1740

aaaaaaaaaa aaaaaaaa 1758

<210> 2
<211> 1758
<212> RNA
<213> Artificial Sequence

<220>
<223> Photinus pyralis luciferase mRNA G/C-enriched

<400> 2
gggagaugua caaagcuuac cauggaggac gccaagaaca ucaagaaggg ccccgccccg 60

uucuaccccc uggaggacgg gaccgcgggc gagcagcucc acaaggccau gaagcgguac 120

gcccuggugc ccgggaccau cgccuucacg gacgcccaca ucgaggucga caucaccuac 180

gcggaguacu ucgagaugag cgugcgccug gccgaggcca ugaagcggua cggccucaac 240

accaaccacc gcaucguggu cugcuccgag aacagccugc aguucuucau gcccgugcug 300

ggggcccucu ucaucggcgu ggcggucgcc ccggccaacg acaucuacaa cgagcgggag 360

cugcugaacu ccaugggcau cagccagccc accguggugu ucgucuccaa gaaggggcuc 420

cagaagaucc ugaacgugca gaagaagcug ccgaucaucc agaagaucau caucauggac 480

agcaagacgg acuaccaggg cuuccagucc auguauaccu ucgugaccag ccaccucccc 540

ccgggguuca acgaguacga cuucgucccc gaguccuucg accgcgacaa gaccaucgcc 600

cugaucauga acagcuccgg cagcacgggg cugcccaagg gcguggcccu cccccaccgg 660

accgcgugcg ugcgcuucuc ccacgcccgg gacccgaucu ucggcaacca gaucaucccc 720

gacaccgcca uccugagcgu cgugcccuuc caccacgggu ucggcauguu caccacgcug 780

ggguaccuca ucugcggcuu ccgcgugguc cugauguacc gguucgagga ggagcuguuc 840

cuccgcuccc ugcaggacua caagauccag agcgcccugc ucgugcccac ccuguucucc 900

uucuucgcca agagcacccu gaucgacaag uacgaccucu ccaaccugca cgagaucgcg 960

agcggcgggg ccccgcugag caaggaggug ggcgaggccg ucgccaagcg guuccaccuc 1020

cccgggaucc gccagggcua cgggcugacc gagacgaccu ccgccauccu gaucaccccc 1080

gagggcgacg acaagcccgg cgcggugggg aagguggucc cguucuucga ggccaaggug 1140

gucgaccucg acaccggcaa gacgcugggg gugaaccagc ggggcgagcu gugcgugcgc 1200

gggcccauga ucaugagcgg cuacgucaac aaccccgagg ccaccaacgc ccucaucgac 1260

aaggacggcu ggcugcacuc cggggacauc gccuacuggg acgaggacga gcacuucuuc 1320

aucguggacc ggcugaagag ccucaucaag uacaagggcu accagguggc gcccgccgag 1380

cuggagucca uccugcucca gcacccgaac aucuucgacg ccggggucgc cggccugccc 1440

gacgacgacg cgggggagcu gcccgccgcc gugguggucc ucgagcacgg caagaccaug 1500

accgagaagg agaucgugga cuacguggcc agccagguca cgaccgccaa gaagcugcgc 1560

ggcggggugg uguucgucga cgaggugccc aagggccuga ccgggaagcu ggacgcgcgg 1620

aagauccgcg agauccucau caaggccaag aagggcggca agaucgccgu cugaggacua 1680

guagaucuaa aaaaaaaaaa aaaaaaaaaa aaaaaaaaaa aaaaaaaaaa aaaaaaaaaa 1740

aaaaaaaaaa aaaaaaaa 1758

<210> 3
<211> 1758
<212> RNA
<213> Artificial Sequence

<220>
<223> Photinus pyralis luciferase mRNA C-enriched

<400> 3
gggagaugua caaagcuuac cauggaggac gccaagaaca ucaagaaggg ccccgccccc 60

uucuaccccc ucgaggacgg caccgccggc gagcagcucc acaaggccau gaagcgcuac 120

gcccucgucc ccggcaccau cgccuucacc gacgcccaca ucgaggucga caucaccuac 180

gccgaguacu ucgagauguc cguccgccuc gccgaggcca ugaagcgcua cggccucaac 240

accaaccacc gcaucgucgu cugcuccgag aacucccucc aguucuucau gcccguccuc 300

ggcgcccucu ucaucggcgu cgccgucgcc cccgccaacg acaucuacaa cgagcgcgag 360

cuccucaacu ccaugggcau cucccagccc accgucgucu ucgucuccaa gaagggccuc 420

cagaagaucc ucaacgucca gaagaagcuc cccaucaucc agaagaucau caucauggac 480

uccaagaccg acuaccaggg cuuccagucc auguauaccu ucgucaccuc ccaccucccc 540

cccggcuuca acgaguacga cuucgucccc gaguccuucg accgcgacaa gaccaucgcc 600

cucaucauga acuccuccgg cuccaccggc cuccccaagg gcgucgcccu cccccaccgc 660

accgccugcg uccgcuucuc ccacgcccgc gaccccaucu ucggcaacca gaucaucccc 720

gacaccgcca uccucuccgu cguccccuuc caccacggcu ucggcauguu caccacccuc 780

ggcuaccuca ucugcggcuu ccgcgucguc cucauguacc gcuucgagga ggagcucuuc 840

cuccgcuccc uccaggacua caagauccag uccgcccucc ucguccccac ccucuucucc 900

uucuucgcca aguccacccu caucgacaag uacgaccucu ccaaccucca cgagaucgcc 960

uccggcggcg ccccccucuc caaggagguc ggcgaggccg ucgccaagcg cuuccaccuc 1020

cccggcaucc gccagggcua cggccucacc gagaccaccu ccgccauccu caucaccccc 1080

gagggcgacg acaagcccgg cgccgucggc aaggucgucc ccuucuucga ggccaagguc 1140

gucgaccucg acaccggcaa gacccucggc gucaaccagc gcggcgagcu cugcguccgc 1200

ggccccauga ucauguccgg cuacgucaac aaccccgagg ccaccaacgc ccucaucgac 1260

aaggacggcu ggcuccacuc cggcgacauc gccuacuggg acgaggacga gcacuucuuc 1320

aucgucgacc gccucaaguc ccucaucaag uacaagggcu accaggucgc ccccgccgag 1380

cucgagucca uccuccucca gcaccccaac aucuucgacg ccggcgucgc cggccucccc 1440

gacgacgacg ccggcgagcu ccccgccgcc gucgucgucc ucgagcacgg caagaccaug 1500

accgagaagg agaucgucga cuacgucgcc ucccagguca ccaccgccaa gaagcuccgc 1560

ggcggcgucg ucuucgucga cgaggucccc aagggccuca ccggcaagcu cgacgcccgc 1620

aagauccgcg agauccucau caaggccaag aagggcggca agaucgccgu cugaggacua 1680

guagaucuaa aaaaaaaaaa aaaaaaaaaa aaaaaaaaaa aaaaaaaaaa aaaaaaaaaa 1740

aaaaaaaaaa aaaaaaaa 1758

<210> 4
<211> 1746
<212> RNA
<213> Artificial Sequence

<220>
<223> Photinus pyralis luciferase G/C-enriched

<400> 4
gggagaaagc uuaccaugga ggacgccaag aacaucaaga agggccccgc ccccuucuac 60

ccccuggagg acgggaccgc cggcgagcag cuccacaagg ccaugaagcg guacgcccug 120

gugcccggga ccaucgccuu caccgacgcc cacaucgagg ucgacaucac cuacgccgag 180

uacuucgaga ugagcgugcg ccuggccgag gccaugaagc gguacggccu caacaccaac 240

caccgcaucg uggucugcuc cgagaacagc cugcaguucu ucaugcccgu gcugggggcc 300

cucuucaucg gcguggccgu cgcccccgcc aacgacaucu acaacgagcg ggagcugcug 360

aacuccaugg gcaucagcca gcccaccgug guguucgucu ccaagaaggg gcuccagaag 420

auccugaacg ugcagaagaa gcugcccauc auccagaaga ucaucaucau ggacagcaag 480

accgacuacc agggcuucca guccauguau accuucguga ccagccaccu cccucccggg 540

uucaacgagu acgacuucgu ccccgagucc uucgaccgcg acaagaccau cgcccugauc 600

augaacagcu ccggcagcac cgggcugccc aagggcgugg cccuccccca ccggaccgcc 660

ugcgugcgcu ucucccacgc ccgggacccc aucuucggca accagaucau ccccgacacc 720

gccauccuga gcgucgugcc cuuccaccac ggguucggca uguucaccac ccuggguuac 780

cucaucugcg gcuuccgcgu gguccugaug uaccgguucg aggaggagcu guuccuccgc 840

ucccugcagg acuacaagau ccagagcgcc cugcucgugc ccacccuguu cuccuucuuc 900

gccaagagca cccugaucga caaguacgac cucuccaacc ugcacgagau cgccagcggc 960

ggggccccuc ugagcaagga ggugggcgag gccgucgcca agcgguucca ccuccccggg 1020

auccgccagg gcuacgggcu gaccgagacc accuccgcca uccugaucac ccccgagggc 1080

gacgacaagc ccggcgccgu ggggaaggug guccccuucu ucgaggccaa gguggucgac 1140

cucgacaccg gcaagacccu gggggugaac cagcggggcg agcugugcgu gcgcgggccc 1200

augaucauga gcggcuacgu caacaacccc gaggccacca acgcccucau cgacaaggac 1260

ggcuggcugc acuccgggga caucgccuac ugggacgagg acgagcacuu cuucaucgug 1320

gaccggcuga agagccucau caaguacaag ggcuaccagg uggcccccgc cgagcuggag 1380

uccauccugc uccagcaccc caacaucuuc gacgccgggg ucgccggccu gcccgacgac 1440

gacgccgggg agcugcccgc cgccguggug guccucgagc acggcaagac caugaccgag 1500

aaggagaucg uggacuacgu ggccagccag gucaccaccg ccaagaagcu gcgcggcggg 1560

gugguguucg ucgacgaggu gcccaagggc cugaccggga agcuggacgc ccggaagauc 1620

cgcgagaucc ucaucaaggc caagaagggc ggcaagaucg ccgucugagg acuaguagau 1680

cuaaaaaaaa aaaaaaaaaa aaaaaaaaaa aaaaaaaaaa aaaaaaaaaa aaaaaaaaaa 1740

aaaaaa 1746

<210> 5
<211> 1745
<212> RNA
<213> Artificial Sequence

<220>
<223> Photinus pyralis luciferase C-enriched

<400> 5
ggagaaagcu uaccauggag gacgccaaga acaucaagaa gggccccgcc cccuucuacc 60

cccucgagga cggcaccgcc ggcgagcagc uccacaaggc caugaagcgc uacgcccucg 120

uccccggcac caucgccuuc accgacgccc acaucgaggu cgacaucacc uacgccgagu 180

acuucgagau guccguccgc cucgccgagg ccaugaagcg cuacggccuc aacaccaacc 240

accgcaucgu cgucugcucc gagaacuccc uccaguucuu caugcccguc cucggcgccc 300

ucuucaucgg cgucgccguc gcccccgcca acgacaucua caacgagcgc gagcuccuca 360

acuccauggg caucucccag cccaccgucg ucuucgucuc caagaagggc cuccagaaga 420

uccucaacgu ccagaagaag cuccccauca uccagaagau caucaucaug gacuccaaga 480

ccgacuacca gggcuuccag uccauguaua ccuucgucac cucccaccuc ccccccggcu 540

ucaacgagua cgacuucguc cccgaguccu ucgaccgcga caagaccauc gcccucauca 600

ugaacuccuc cggcuccacc ggccucccca agggcgucgc ccucccccac cgcaccgccu 660

gcguccgcuu cucccacgcc cgcgacccca ucuucggcaa ccagaucauc cccgacaccg 720

ccauccucuc cgucgucccc uuccaccacg gcuucggcau guucaccacc cucggcuacc 780

ucaucugcgg cuuccgcguc guccucaugu accgcuucga ggaggagcuc uuccuccgcu 840

cccuccagga cuacaagauc caguccgccc uccucguccc cacccucuuc uccuucuucg 900

ccaaguccac ccucaucgac aaguacgacc ucuccaaccu ccacgagauc gccuccggcg 960

gcgccccccu cuccaaggag gucggcgagg ccgucgccaa gcgcuuccac cuccccggca 1020

uccgccaggg cuacggccuc accgagacca ccuccgccau ccucaucacc cccgagggcg 1080

acgacaagcc cggcgccguc ggcaaggucg uccccuucuu cgaggccaag gucgucgacc 1140

ucgacaccgg caagacccuc ggcgucaacc agcgcggcga gcucugcguc cgcggcccca 1200

ugaucauguc cggcuacguc aacaaccccg aggccaccaa cgcccucauc gacaaggacg 1260

gcuggcucca cuccggcgac aucgccuacu gggacgagga cgagcacuuc uucaucgucg 1320

accgccucaa gucccucauc aaguacaagg gcuaccaggu cgcccccgcc gagcucgagu 1380

ccauccuccu ccagcacccc aacaucuucg acgccggcgu cgccggccuc cccgacgacg 1440

acgccggcga gcuccccgcc gccgucgucg uccucgagca cggcaagacc augaccgaga 1500

aggagaucgu cgacuacguc gccucccagg ucaccaccgc caagaagcuc cgcggcggcg 1560

ucgucuucgu cgacgagguc cccaagggcc ucaccggcaa gcucgacgcc cgcaagaucc 1620

gcgagauccu caucaaggcc aagaagggcg gcaagaucgc cgucugagga cuaguagauc 1680

uaaaaaaaaa aaaaaaaaaa aaaaaaaaaa aaaaaaaaaa aaaaaaaaaa aaaaaaaaaa 1740

aaaaa 1745

<210> 6
<211> 2035
<212> RNA
<213> Artificial Sequence

<220>
<223> Photinus pyralis luciferase G/C-enriched

<400> 6
ggggcgcugc cuacggaggu ggcagccauc uccuucucgg caucaagcuu accauggagg 60

acgccaagaa caucaagaag ggccccgccc cguucuaccc ccuggaggac gggaccgcgg 120

gcgagcagcu ccacaaggcc augaagcggu acgcccuggu gcccgggacc aucgccuuca 180

cggacgccca caucgagguc gacaucaccu acgcggagua cuucgagaug agcgugcgcc 240

uggccgaggc caugaagcgg uacggccuca acaccaacca ccgcaucgug gucugcuccg 300

agaacagccu gcaguucuuc augcccgugc ugggggcccu cuucaucggc guggcggucg 360

ccccggccaa cgacaucuac aacgagcggg agcugcugaa cuccaugggc aucagccagc 420

ccaccguggu guucgucucc aagaaggggc uccagaagau ccugaacgug cagaagaagc 480

ugccgaucau ccagaagauc aucaucaugg acagcaagac ggacuaccag ggcuuccagu 540

ccauguauac cuucgugacc agccaccucc ccccgggguu caacgaguac gacuucgucc 600

ccgaguccuu cgaccgcgac aagaccaucg cccugaucau gaacagcucc ggcagcacgg 660

ggcugcccaa gggcguggcc cucccccacc ggaccgcgug cgugcgcuuc ucccacgccc 720

gggacccgau cuucggcaac cagaucaucc ccgacaccgc cauccugagc gucgugcccu 780

uccaccacgg guucggcaug uucaccacgc ugggguaccu caucugcggc uuccgcgugg 840

uccugaugua ccgguucgag gaggagcugu uccuccgcuc ccugcaggac uacaagaucc 900

agagcgcccu gcucgugccc acccuguucu ccuucuucgc caagagcacc cugaucgaca 960

aguacgaccu cuccaaccug cacgagaucg cgagcggcgg ggccccgcug agcaaggagg 1020

ugggcgaggc cgucgccaag cgguuccacc uccccgggau ccgccagggc uacgggcuga 1080

ccgagacgac cuccgccauc cugaucaccc ccgagggcga cgacaagccc ggcgcggugg 1140

ggaagguggu cccguucuuc gaggccaagg uggucgaccu cgacaccggc aagacgcugg 1200

gggugaacca gcggggcgag cugugcgugc gcgggcccau gaucaugagc ggcuacguca 1260

acaaccccga ggccaccaac gcccucaucg acaaggacgg cuggcugcac uccggggaca 1320

ucgccuacug ggacgaggac gagcacuucu ucaucgugga ccggcugaag agccucauca 1380

aguacaaggg cuaccaggug gcgcccgccg agcuggaguc cauccugcuc cagcacccga 1440

acaucuucga cgccgggguc gccggccugc ccgacgacga cgcgggggag cugcccgccg 1500

ccgugguggu ccucgagcac ggcaagacca ugaccgagaa ggagaucgug gacuacgugg 1560

ccagccaggu cacgaccgcc aagaagcugc gcggcggggu gguguucguc gacgaggugc 1620

ccaagggccu gaccgggaag cuggacgcgc ggaagauccg cgagauccuc aucaaggcca 1680

agaagggcgg caagaucgcc gucugaggac uagugcauca cauuuaaaag caucucagcc 1740

uaccaugaga auaagagaaa gaaaaugaag aucaauagcu uauucaucuc uuuuucuuuu 1800

ucguuggugu aaagccaaca cccugucuaa aaaacauaaa uuucuuuaau cauuuugccu 1860

cuuuucucug ugcuucaauu aauaaaaaau ggaaagaacc uagaucuaaa aaaaaaaaaa 1920

aaaaaaaaaa aaaaaaaaaa aaaaaaaaaa aaaaaaaaaa aaaaaaaaaa augcaucccc 1980

cccccccccc cccccccccc cccccccaaa ggcucuuuuc agagccacca gaauu 2035

<210> 7

<211>  2035
<212>  RNA
<213>  Artificial Sequence

<220>
<223>  Photinus pyralis luciferase C-enriched

<400>  7
ggggcgcugc cuacggaggu ggcagccauc uccuucucgg caucaagcuu accauggagg      60

acgccaagaa caucaagaag ggccccgccc ccuucuaccc ccucgaggac ggcaccgccg     120

gcgagcagcu ccacaaggcc augaagcgcu acgcccucgu ccccggcacc aucgccuuca     180

ccgacgccca caucgagguc gacaucaccu acgccgagua cuucgagaug uccguccgcc     240

ucgccgaggc caugaagcgc uacggccuca acaccaacca ccgcaucguc gucugcuccg     300

agaacucccu ccaguucuuc augcccgucc ucggcgcccu cuucaucggc gucgccgucg     360

cccccgccaa cgacaucuac aacgagcgcg agcuccucaa cuccaugggc aucucccagc     420

ccaccgucgu cuucgucucc aagaagggcc uccagaagau ccucaacguc cagaagaagc     480

uccccaucau ccagaagauc aucaucaugg acuccaagac cgacuaccag ggcuuccagu     540

ccauguauac cuucgucacc ucccaccucc cccccggcuu caacgaguac gacuucgucc     600

ccgaguccuu cgaccgcgac aagaccaucg cccucaucau gaacuccucc ggcuccaccg     660

gccuccccaa gggcgucgcc cucccccacc gcaccgccug cguccgcuuc ucccacgccc     720

gcgaccccau cuucggcaac cagaucaucc ccgacaccgc cauccucucc gucguccccu     780

uccaccacgg cuucggcaug uucaccaccc ucggcuaccu caucugcggc uuccgcgucg     840

uccucaugua ccgcuucgag gaggagcucu uccuccgcuc ccuccaggac uacaagaucc     900

aguccgcccu ccucgucccc acccucuucu ccuucuucgc caaguccacc cucaucgaca     960

aguacgaccu cuccaaccuc cacgagaucg ccuccggcgg cgccccccuc uccaaggagg    1020

ucggcgaggc cgucgccaag cgcuuccacc uccccggcau ccgccagggc uacggccuca    1080

ccgagaccac cuccgccauc cucaucaccc ccgagggcga cgacaagccc ggcgccgucg    1140

gcaaggucgu ccccuucuuc gaggccaagg ucgucgaccu cgacaccggc aagacccucg    1200

gcgucaacca gcgcggcgag cucugcgucc gcggccccau gaucaugucc ggcuacguca    1260

acaaccccga ggccaccaac gcccucaucg acaaggacgg cuggcuccac uccggcgaca    1320

ucgccuacug ggacgaggac gagcacuucu ucaucgucga ccgccucaag ucccucauca    1380

aguacaaggg cuaccagguc gcccccgccg agcucgaguc cauccuccuc cagcacccca    1440

acaucuucga cgccggcguc gccggccucc ccgacgacga cgccggcgag cuccccgccg    1500

ccgucgucgu ccucgagcac ggcaagacca ugaccgagaa ggagaucguc gacuacgucg    1560

ccucccaggu caccaccgcc aagaagcucc gcggcggcgu cgucuucguc gacgaggucc    1620

ccaagggccu caccggcaag cucgacgccc gcaagauccg cgagauccuc aucaaggcca    1680

agaagggcgg caagaucgcc gucugaggac uagugcauca cauuuaaaag caucucagcc 1740

uaccaugaga auaagagaaa gaaaaugaag aucaauagcu uauucaucuc uuuuucuuuu 1800

ucguuggugu aaagccaaca cccugucuaa aaaacauaaa uuucuuuaau cauuuugccu 1860

cuuuucucug ugcuucaauu aauaaaaaau ggaaagaacc uagaucuaaa aaaaaaaaaa 1920

aaaaaaaaaa aaaaaaaaaa aaaaaaaaaa aaaaaaaaaa aaaaaaaaaa augcaucccc 1980

cccccccccc cccccccccc cccccccaaa ggcucuuuuc agagccacca gaauu 2035

<210> 8
<211> 18
<212> DNA
<213> Artificial Sequence

<220>
<223> T7 RNA polymerase promoter sequence

<400> 8
taatacgact cactatag 18

<210> 9
<211> 18
<212> DNA
<213> Artificial Sequence

<220>
<223> T3 RNA polymerase promoter sequence

<400> 9
aattaaccct cactaaag 18

<210> 10
<211> 18
<212> DNA
<213> Artificial Sequence

<220>
<223> SP6 RNA polymerase promoter sequence

<400> 10
atttaggtga cactatag 18

<210> 11
<211> 13
<212> RNA
<213> Artificial Sequence

<220>
<223> Kozak sequence

<400> 11
gccgccacca ugg 13

<210> 12

<211>  14
<212>  RNA
<213>  Artificial Sequence

<220>
<223>  stabilization sequence consensus


<220>
<221>  misc_feature
<222>  (5)..(5)
<223>  n can be 0 - 5 nucleotides, n is a, g , c or t

<220>
<221>  misc_feature
<222>  (10)..(10)
<223>  y can be 0 - 4 pyrimidines, y is t/u or c

<400>  12
yccancccwy cycc                                                    14

## Claims

1. Method for targeted modulation, preferably reduction, of the immune response against an mRNA coding for at least one biologically active polypeptide or protein, wherein the method comprises the steps of
(1) identifying a target mRNA wild type sequence coding for the biologically active polypeptide or protein and
(2) modifying at least 70% of the codons of the wild type sequence which are cytosine content optimizable by increasing the cytosine-content of the mRNA such that the cytosine-content of the region of the coding region of the modified mRNA is larger than the cytosine-content of the coding region of the wild type mRNA, whereby the encoded amino acid sequence is unchanged compared to the wild type sequence and, optionally
(3) modifying at least 70% of the codons for amino acids which are not eligible for C-optimization, but are guanosine-content optimizable by increasing the guanosine-content

2. Method according to claim 1, wherein the coding region of the target mRNA is modified such that a maximal cytosine-content is achieved by introduction of codons, which encode relatively frequent tRNAs.

3. Method according to claim 1 or claim 2, wherein the cytosine-content of the region of the modified mRNA coding for at least one the polypeptide or protein is at least 10%, preferably at least 12.5%, more preferred at least 15% greater than the cytosine-content compared to the polypeptide or protein coding region of the wild type mRNA.

4. Method according to any one of claims 1 - 3, wherein all codons of the wild type sequence, which are not cytosine-content optimizable and which code for a relatively rare codon in the cell have been replaced by codons which code for a relatively frequent tRNA in the cell, which carries the same amino acid as the relatively rare tRNA.

5. Method according to any one of claims 1 - 4, wherein the codon adaptation index (CAI) of the coding region of the modified target mRNA is greater by at least 0.05, preferably greater by at least 0.1, preferably greater by at least 0.125, more preferred greater by at least 0.15 than the CAI of the wild type region of the mRNA coding for the polypeptide or protein.

6. Method according to any one of claims 1 - 5, wherein the method further comprises the step of determining the immunogenicity and/or immunostimulatory capacity of the modified target mRNA coding for the at least one polypeptide or protein.

7. Method according to any one of claims 1 - 6, wherein determining the immunogenicity and/or immunostimulatory capacity of the modified target mRNA comprises the sub-steps of
(i) transfecting PBMC with the modified mRNA according to any one of claim 1 - 5,
(ii) cultivating the cells for an appropriate amount of time, preferably at least 8 hours, preferably for at least 12h, more preferably for at least 20 hours,
(iii) measuring the amount of pro-inflammatory cytokines in the cell supernatant.

8. Method according to any one of claims 1 - 7, wherein the target mRNA of lower immunogenicity and/or immunostimulatory capacity coding for the polypeptide or protein is selected, optionally the method according to any one of claims 1 - 7 is reiterated to further decrease the immunogenicity and/or immunostimulatory capacity of the target mRNA.

9. Method according to any one of claims 1 - 8, wherein the method of targeted modulation of the immune response against a target mRNA encoding at least one biologically active polypeptide or protein is carried out by way of executing at least one algorithm on a computer with the aid of suitable software.

10. Method according to any one of claims 1 -9, wherein the modified target mRNA is obtained by synthesis.

11. Method according to any one of claims 1- 10, wherein the modified target mRNA is obtained by in vitro transcription, preferably by bacteriophage polymerase-mediated in vitro transcription, preferably by Sp6 polymerase in vitro transcription and/or T3 polymerase-mediated in vitro transcription, more preferably by T7 polymerase-mediated in vitro transcription.

12. Modified target mRNA encoding at least one biologically active polypeptide or protein obtainable by any one of claims 1 - 11 having reduced immunogenicity and/or immunostimulatory capacity compared to the wild type target mRNA.

13. Modified target mRNA according to claim 12, wherein the modified target mRNA is **characterized by** a lower affinity to one or more of TLR3, TLR7, TLR8, PKR, MDA5, RIG-I, LGP2 or 2'-5'-oligoadenylate synthetase compared to the wild type target mRNA encoding the at least one biologically active polypeptide or protein.

14. Modified target mRNA according to one of claims 12 or 13, wherein the modified target mRNA is devoid of destabilising sequence elements (DSE) in the 3' and/or 5' UTR.

15. Modified target mRNA according to any one of claims 12 - 14, wherein the modified target mRNA further comprises a 5' CAP structure and/or a polyA-tail of at least 60 nucleotides, preferably of at least 70 nucleotides and/or a 3' stabilizing sequence.

16. Pharmaceutical composition comprising the modified target mRNA obtainable by any one of claims 1 to 11 and/or the modified target mRNA according to any one of claims 12- 15, optionally comprising one or more pharmaceutically acceptable excipients, carriers, diluents and/or vehicles.

17. Pharmaceutical composition according to claim 16 for use in the treatment of protein deficiency disorders by protein replacement therapy.

18. Pharmaceutical composition according to claim 16 or the modified target mRNA according to any one of claims 12 - 15 for use in the treatment of diseases amenable to protein replacement therapy, preferably for use in the treatment of e.g. hereditary or endocrinological dieases, selected from the group comprising amino acid metabolism disorders, carbohydrate metabolism disorders, cholesterol biosynthesis disorders, fatty acid oxidation defects and fat metabolism disorders, lactic acidosis, glycogen storage diseases, mitochondrial disorders, organic acid disorders, urea cycle disorders, vitamin/cofactor disorders, lysosomal storage disease disorders.

19. The pharmaceutical composition according to claim 16 or the modified target mRNA according to any one of claims 12 to 15 for use in the treatment of a patient by using a method for expressing a biologically active polypeptide or protein in a tissue *in vivo,* the method comprising contacting the patient with the pharmaceutical composition or contacting the tissue with the modified target mRNA, wherein administering the pharmaceutical composition or the modified target mRNA results in a reduced innate immune response by the patient relative to a patient contacted with the wild type target mRNA molecule encoding the same polypeptide or protein, wherein the level of mRNA expression *in vivo* is preferably increased by the modified target mRNA of the invention as compared to the wild type target mRNA.
